(19) Europäisches Patentamt
European Patent Office
Office européen des brevets

(11) EP 0 832 976 B1

(12) EUROPEAN PATENT SPECIFICATION

(45) Date of publication and mention of the grant of the patent:
**04.06.2003 Bulletin 2003/23**

(21) Application number: **97116478.5**

(22) Date of filing: **22.09.1997**

(51) Int Cl.[7]: **C12N 15/54**, C12N 15/62, C12N 9/12, C12N 5/18, C12N 1/21, C07K 16/40, C12P 21/08

(54) **Thermostable DNA polymerase from Bacillus pallidus**

Thermostabile DNA Polymerase aus Bacillus pallidus

ADN polymérase thermostable de Bacillus pallidus

(84) Designated Contracting States:
**DE FR**

(30) Priority: **23.09.1996 US 717515**
**26.08.1997 US 918416**

(43) Date of publication of application:
**01.04.1998 Bulletin 1998/14**

(73) Proprietor: **Becton, Dickinson and Company**
**Franklin Lakes, New Jersey 07417 (US)**

(72) Inventor: **Hamilton, Paul T.**
**Cary, North Carolina 27513 (US)**

(74) Representative: **Gerbino, Angelo**
**Jacobacci & Partners S.p.A.**
**Corso Regio Parco, 27**
**10152 Torino (IT)**

(56) References cited:
**EP-A- 0 517 418**
**EP-A- 0 684 315**
**WO-A-95/14782**
**WO-A-95/27067**
**US-A- 5 002 875**

- **T. SCHOLZ ET AL.: "Bacillus pallidus sp. nov., a new thermophilic species from sewage" SYSTEM. APPL. MICROBIOL, vol. 9, 1987, FISCHER, STUTTGART, FRG, pages 91-96, XP002055609**
- **SELLMANN E ET AL: "PURIFICATION AND CHARACTERIZATION OF DNA POLYMERASES FROM BACILLUS SPECIES" JOURNAL OF BACTERIOLOGY, vol. 174, no. 13, July 1992, pages 4350-4355, XP000611611**
- **RIGGS M G ET AL: "CONSTRUCTION OF SINGLE AMINO ACID SUBSTITUTION MUTANTS OF CLONED BACILLUS STEAROTHERMOPHILUS DNA POLYMERASE I WHICH LACK 5' - 3' EXONUCLEASE ACTIVITY" BIOCHIMICA ET BIOPHYSICA ACTA. GENE STRUCTURE AND EXPRESSION, vol. 1307, no. 2, 1996, pages 178-186, XP000611819**
- **HAMILTON P T ET AL: "Bacillus pallidus DNA polymerase I: Molecular cloning, nucleotide sequence, expression, and purification." 97TH GENERAL MEETING OF THE AMERICAN SOCIETY FOR MICROBIOLOGY, MIAMI BEACH, FLORIDA, USA, MAY 4-8, 1997. ABSTRACTS OF THE GENERAL MEETING OF THE AMERICAN SOCIETY FOR MICROBIOLOGY 97 (0). 1997. 287. ISSN: 1060-2011, XP002055610**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 832 976 B1

## Description

### FIELD OF THE INVENTION

The invention relates to DNA polymerases, genes coding for DNA polymerases, production of recombinant DNA polymerases. Also provided are antibodies to DNA polymerases and methods of using these antibodies

### BACKGROUND OF THE INVENTION

Type I DNA polymerases (Pol I) are ubiquitous enzymes in Eubacteria. They are multifunctional/multidomain enzymes which appear to be involved in DNA repair and DNA replication. The *E. coli* Pol I, and most other Type I DNA polymerases characterized to date, have three enzymatic activities: DNA polymerization (5'-3'), 3'-5' exonuclease activity and 5'-3' exonuclease activity. Each of these activities has been localized to a particular region or domain of the protein. In *E. coli* Pol I, the N-terminal domain (amino acids 1-324) encodes the 5'-3' exonuclease activity, the central domain (amino acids 324-517) encodes the 3'-5' exonuclease activity and the C-terminal domain (amino acids 521-928) encodes the DNA polymerase activity. When *E. coli* Pol I is cleaved into two fragments by subtilisin digestion, the larger fragment (Klenow fragment) has 3'-5' exonuclease and DNA polymerase activities and the smaller fragment has 5'-3' exonuclease activity.

DNA polymerase I has been isolated, cloned and sequenced from several eubacterial species, including *Streptococcus pneumoniae* (A. Diaz, et al. 1992. J. Bacteriol. 174:2014-2024), *Bacillus stearothermophilus* (S-M. Phang, et al. 1995. Gene 163:65-68 and WO 95/27067), *Bacillus caldotenax* (T. Uemori, et al. 1993. J. Biochem. 113: 401-410), and *Thermus aquaticus* (Lawyer et al. 1993. PCR Methods and Applications. 2:275-287). The temperature optimum for activity of the *E. coli* and *S. pneumoniae* Pol I enzymes is about 37°C, i.e., they are mesophilic. In contrast, the polymerases of bacteria with higher temperature tolerance have higher temperature optima - about 60-70°C for the *Bacillus* species and about 80°C for *T. aquaticus*. These polymerases are thermophilic and may be useful at the temperatures of PCR and thermophilic SDA.

Alignment of the amino acid sequences of DNA polymerase I from eubacterial and bacteriophage sources has shown conserved motifs in both the 5'-3' exonuclease domain and the DNA polymerase domain. These conserved sequences typically represent amino acids which are important for the structure and/or function of the enzyme. Based on knowledge of conserved sequences, "Klenow-like" forms of the polymerases of species other than *E. coli* (i.e., forms lacking the 5'-3' exonuclease activity) have also been reported. Absence of 5'-3' exonuclease activity is particularly important for use of the polymerases in Strand Displacement Amplification (SDA - G. T. Walker, et al. 1992. *Proc. Natl. Acad. Sci. USA* **89**, 392-396; G. T. Walker, et al. 1992. *Nuc. Acids. Res.* **20**, 1691-1696; U.S. Patent No. 5,455,166; U.S. Patent No. 5,270,184; EP 0 684 315), as the SDA polymerase must lack 5'-3' exonuclease activity, either naturally or by genetic manipulation, to prevent digestion of the strand downstream of the nick. For SDA, the polymerase must also incorporate the derivatized deoxynucleoside triphosphates (dNTPs) required for amplification (nucleotide analogs such as αthio-dNTPs) and displace a downsteam single strand from a double stranded molecule starting at a single stranded nick. It is also desirable, but not required, that the polymerase be capable of incorporating dUTP to allow amplicon decontamination.

### SUMMARY OF THE INVENTION

A novel DNA polymerase I has been identified in *Bacillus pallidus* (Bpa Pol I) and the gene encoding this Pol I has been cloned, sequenced and expressed to produce the polymerase. The full-length wild-type enzyme has thermophilic DNA polymerase activity and thermophilic 5'-3' exonuclease activity. A Klenow-like deletion which inactivates the 5'-3' exonuclease activity has been constructed and expressed to produce a polymerase which is particularly useful in SDA. Bpa Pol I may also be used to immunize animals for production of antibodies which bind to Bpa Pol I. The specific subject-matter of the invention is indicated in the appended claims.

### DETAILED DESCRIPTION OF THE INVENTION

Initially, the "Klenow-like" fragment of Bpa Pol was cloned using multiple sequence alignments of DNA Pol I genes of other species available in the GenBank sequence database to design degenerate PCR primers based on relatively conserved sequences within the 5'-3' exonuclease domain and at the C-terminal end of the polymerase gene. The primers were designed to include restriction enzyme recognition sites which allowed cloning and expression of amplification products in the pBLUESCRIPT plasmid (Stratagene). The forward primer was designed based on alignment of the *B. caldotenax*, *B. stearothermophilus*, *E. coli* and *S. pneumoniae* Pol I genes. The reverse primer was designed based on alignment of the *B. caldotenax, B. stearothermophilus* and *S. pneumoniae* Pol I genes. Two gene sequences for *B. stearothermophilus* were listed in GenBank, and both were used in the sequence alignments.

The degenerate PCR primers were as follows:

SEQ ID NO:1

TGCCAATCTAGAAGGCGTGCCSGGCRTCGGKRAAAARAC

(XbaI site for cloning is underlined)

SEQ ID NO:2

CACCAAGGATCCSYTTTTATTTSGCGTCRTACCAYGT

(BamHI site for cloning is underlined)

*Bacillus pallidus* (ATCC#51176) was grown in the ATCC recommended culture medium at 55°C and the genomic DNA was isolated using conventional techniques. Using the degenerate primers SEQ ID NO: 1 and SEQ ID NO:2, a 2 Kb segment of the genome was amplified. This segment was cloned in the pBLUESCRIPT vector and transformed into *E. coli.* The gene product was expressed in the transformants by induction with IPTG as recommended by the manufacturer of the cloning vector. Cell-free lysates were prepared and assayed for polymerase activity by incorporation of $^{32}$P-labeled nucleotides into activated calf thymus DNA (Pharmacia), as follows. The polymerase was serially diluted at room temperature in freshly prepared enzyme diluent (25 mM $K_iPO_4$ pH 7.5, 50 mM ammonium sulfate, 10 mM 2-mercaptoethanol, 1 mg/ml BSA) and mixed on a vortex mixer. Ninety µL of reaction buffer (25 mM $K_iPO_4$ pH 7.5, 0.15 mM each dNTP, 4 mM $MgCl_2$, 4.5 µg/reaction activated calf thymus DNA, 0.3 mg/reaction activated calf thymus DNA, 0.3 µL per reaction 3000 mCu/mmol $\alpha^{32}$-P-dCTP) was added to each reaction tube and prewarmed for 5 minutes at the selected reaction temperature. After prewarming, 10 µL of the polymerase dilution was added and mixed on a vortex mixer. Blank reactions had only 10 µL of the enzyme diluent added. The reactions were incubated for 15 minutes at the selected reaction temperature, removing 15 µL aliquots at 2, 5, 10 and 15 minutes and adding them to 45 µL of 25 mM EDTA to stop the reaction. Similar time points were taken from the blank control. After all time points were taken, 40 µL of the terminated reaction was spotted onto a DE-81 filter disk (representing 10 µL of the original reaction). The filters were washed at least four times in 0.3 M ammonium formate pH 8.0 (5 minutes each wash) using 10 mL per filter. Washing was performed by dropping the spotted filter into a beaker of ammonium formate and agitating gently every 2-3 minutes The waste was decanted and additional aliquots of the wash buffer were poured onto the filters. Following the last wash the filters were rinsed in methanol and placed on Whatman paper to dry for about 5 minutes. The filters were then placed in scintillation vials and counted. An increase in radioactivity retained by the filter indicated that the recombinant expression product of the transformant exhibited polymerase activity. Polymerase activity, wherein one unit (U) is defined as the amount required to incorporate 10 nmole of total nucleotide into acid insoluble form in 30 min., was calculated according to the following equation:

U/mL = (net cpm) (nmole/cpm) (1 u/10 nmole) (100% dNTP/x%dXTP) (1/0.01 mL enz added) (dilution factor)

wherein

net cpm = total cpm incorporated at 30 min. point for total reaction, e.g., for the 100 µL reactions described above after 30 min., net cpm = (time point cpm) (10 µLcounted) (30 min./10 min. time point)

nmole/cpm = specific activity of reaction

100% dNTP/x% dXTP = the ratio of total nucleotides to %G or C, or the ratio of total nucleotides to %A or T in the template DNA. Calf thymus DNA is about 22% G or C and 28% A or T. The value selected corresponds to the $^{32}$-P labeled nucleotide employed.

Specific activity of the reaction was typically determined by spotting 5 µL of the blank reaction (90 µL buffer

mix + 10 μL enzyme diluent) onto each of three DE-81 filters, counting the filters and obtaining the average cpm. Specific activity was calculated as (5 μL)(0.15 nmoles)/average cpm = nmole/cpm.

For example, the unit activity of a thermophilic polymerase was calculated as follows. The polymerase was assayed as described above using 10 μL of a 1:5000 dilution and 10 μL of a 1:10,000 dilution and $\alpha^{32}$P-dATP. Three time points were taken (5 minutes, 10 minutes and 15 minutes) and background counts on the filters as well as blank control reactions were included. The average background count on three filters was 1442 cpm. The average cpm of the blank control (2 μL spotted on each of two filters) was 240,536 cpm, resulting in a specific activity of $1.24 \times 10^{-6}$ nmole/cpm. Units/mL were calculated for each time point of each dilution. As an example, U/mL for the 5 minute time point of the 1:5000 dilution was calculated as follows: net cpm = (3714 cpm-1442 cpm)(10 μL)(30 min./5 min.) = 134,820 net cpm; U/mL = 134,820 net cpm ($1.24 \times 10^{-6}$ nm/cpm)(0.1 U/nm)(3.57)(100)(5000) = 29,841. Units/mL for each time point were averaged for each dilution. The dilution averages were averaged to obtain a combined average U/mL. In this example, the combined average was 26,494 U/mL

To simplify purification of the recombinant polymerase and to obtain higher expression levels, the amplified fragment was recloned into the pMAL-c plasmid (New England BioLabs) as recommended by the manufacturer. These constructs placed the large fragment of the Bpa polymerase under the control of the lac promoter of *E. coli*. Upon induction with IPTG, transformed host cells expressed a fusion product which contained the large fragment of Bpa Pol I fused to the maltose binding protein (MBP, the malE gene product). The coding sequence for this clone (clone MBP/Bpa-192) is shown in SEQ ID NO:5, wherein nucleotides 1-3237 code for the MBP/Bpa fusion protein and nucleotides 3238-3255 code for a polyhistidine affinity purification tag. The MBP portion of the fusion protein allows purification of the expression product on amylose resin. The MBP/Bpa Pol I fusion protein had a molecular weight of about 117 Kd and was expressed as about 5-10% of total cell protein. The deduced amino acid sequence of the fusion protein is shown in SEQ ID NO:6, amino acids 1-1078.

The MBP/Bpa Pol I fusion protein was purified on amylose resin according to the protocol recommended by New England BioLabs. A temperature profile in the DNA polymerase activity assay showed activity between about 30°C and about 75°C with optimal activity at about 65°C. In a primer extension assay in which two adjacent primers were hybridized to a target sequence to stage a "nick", the polymerase was shown to be capable of initiating synthesis from a nick and displacing a downstream primer, indicating utility in SDA. Amplification was demonstrated in a thermophilic SDA reaction (EP 0 684 315), confirming strand displacing ability, the absence of 5'-3' exonuclease activity and an ability to incorporate dNTP analogs. The fewest number of initial targets tested for amplification (1,000) was detectable in the SDA reaction with 3 units of Bpa Pol I and 80 units of BsoBI. A unit of Bpa Pol I activity was defined in the $^{32}$P-incorporation assay described above and corresponds to the amount of polymerase which incorporates 10 nM of nucleotide in 30 min. Reverse transcriptase activity in the presence of $Mn^{2+}$ was demonstrated in an assay in which the polymerase extended a DNA primer annealed to an RNA template.

To isolate the full-length genomic copy of the Bpa Pol I gene, a genomic library of *B. pallidus* DNA was constructed by cloning genomic DNA digested with BclI into a λZAP vector (Stratagene). The library was screened by hybridization to the Bpa Pol I PCR product and a 5.8 Kb DNA fragment containing the Bpa Pol I gene was identified and isolated. The DNA sequence of this fragment was determined by conventional sequencing techniques and was found to contain the full-length coding sequence for Bpa Pol I as well as two open reading frames upstream from the Bpa Pol I gene and two open reading frames downstream from the Bpa Pol I gene. The coding sequence for the full-length Bpa Pol I is shown in SEQ ID NO:3 and the deduced amino acid sequence of the full-length polymerase encoded by SEQ ID NO:3 is shown in SEQ ID NO:4. The large fragment initially cloned, in which the 5'-3' exonuclease domain is deleted, corresponds to amino acids 192-876 of SEQ ID NO:4 and is encoded by nucleotides 574-2628 of SEQ ID NO:3. Amino acid sequence comparison revealed 68% similarity to Bst polymerase *(B. strearothermophilus*, Riggs et al. 1995) and 67% similarity to Bca polymerase (*B. caldotenax,* Uemori et al. 1993). The full-length Bpa Pol I gene was cloned into pMAL-c to produce a MBP fusion protein. This clone was designated MBP/Bpa-1 and was shown to express thermophilic DNA polymerase activity and thermophilic 5'-3' exonuclease activity in the assays previously described. MBP/Bpa-1 expresses the nucleic acid sequence shown in SEQ ID NO:7 to produce Bpa Pol I having the amino acid sequence shown in SEQ ID NO:8. Nucleotides 1-3810 of SEQ ID NO:7 code for the MBP/Bpa fusion protein and nucleotides 3811-3828 code for a polyhistidine affinity purification tag. In SEQ ID NO:8, amino acids 1-1270 represent the MBP/Bpa fusion protein and amino acids 1271-1276 are the added polyhistidine affinity purification tag.

In addition, the large fragment with the 5'-3' exonuclease domain deleted was subcloned into plasmid pTRC99A (Pharmacia) without the MBP purification tag. This clone, designated pTRC/Bpa1 was deposited under the Budapest Treaty with the American Type Culture Collection, Rockville, Maryland, as ATCC Accession No. 98160 on August 30, 1996. The large fragment may be derived from the deposited full-length clone using conventional methods for genetic manipulation such as *in vitro* mutagenesis and cloning. Expression of the polymerase in pTRC/Bpa1 produced an unfused polymerase protein which was tested in SDA as previously described. The polymerase supported amplification, confirming that the 5'-3' exo- activity was inactive and that the unfused polymerase possessed the other activities required for SDA (strand displacing activity, initiation at a nick and incorporation of modified dNTPs).

When no affinity purification tag is present the polymerase protein may be purified, for example, as follows. *E. coli* cells expressing recombinant Bpa Pol I (about 1.28 g of cell paste) were isolated from the culture medium and resuspended in 3 mL of lysis buffer (20 mM imidazole-HCl pH 6.94, 0.2 M KCl, 10% glycerol (w/v), 0.5 mM $Na_2$EDTA, 1 mM DTT) and the cell clumps were broken up using a pipette. The cell suspension was sonicated for 10 minutes in 20 second bursts to lyse the cells and centrifuged to remove cell debris. Of course, when a secretory expression system for the recombinant polymerase is employed lysis of the cells is not necessary. In these systems the cells are separated from the culture medium and the culture medium rather than a cell lysate is processed according to the following protocol to isolate the polymerase. The supernatant of the centrifuged cell lysate was then heated at 55°C for 15 min. in a water bath and centrifuged 15 min. in a microcentrifuge to pellet the denatured proteins. Solid ammonium sulfate was added to the supernatant to 25% saturation. The sample was incubated with gentle agitation at 4°C for 15 min. and microcentrifuged in the cold for 15 min. The supernatant was removed to a new microcentrfuge tube and lysis buffer saturated with ammonium sulfate was added to give the desired % saturation, followed by gentle agitation for 15 min and centrifugation as before. The ammonium sulfate cuts taken were 35%, 45%, 55% and 65% saturation. The pellets of all ammonium sulfate cuts were quick-frozen in a dry ice-ethanol bath and stored at -76°C while polymerase activity assays as described above were performed on samples taken from the lysis and fractionation steps to evaluate the efficiency of the purification process. The 65% ammonium sulfate saturated supernatant was stored on ice during the polymerase assays.

The polymerase activity present in the crude cell extract was stable through the heat treatment, with about 86% of total activity recovered and a 75% reduction in the total protein concentration as estimated by UV absorbance measurements. This corresponds to at least a 3-fold enrichment of the Bpa polymerase in a single simple step. Bpa Pol I appeared to be resistant to precipitation by ammonium sulfate. Most of the activity remained in the 65% saturated supernatant, although about 20% did precipitate and was recovered in the 55% and 65% pellets. Either method may therefore be employed at this step of the purification process, i.e., recovering the polymerase in the pellet at 55% ammonium sulfate or higher, or recovering the polymerase in the supernatant of about a 50-55% ammonium sulfate cut.

The pooled 65% ammonium sulfate supernatant and the resuspended 55% and 65% ammonium sulfate pellets were dialyzed for 4 hours against 750 volumes of Buffer A, diluted 5-fold in 20 mM imidazole-HCl pH 6.94, 20 mM KCl, 10% glycerol (w/v), 0.5 mM $Na_2$EDTA, 1 mM DTT (Buffer A) and loaded at a flow rate of 0.25 mL/min. onto a DEAE cellulose column equilibrated in Buffer A. After sample loading, Buffer A flow was continued at 0.25 mL/min. until UV monitoring of the fluid exiting the column indicated protein was present. At this point, collection of 1 mL fractions was begun and the flow rate was increased to 1 mL/min. The column was washed with Buffer A for 20 min. at this flow rate. A linear gradient (60 mL total gradient volume) from Buffer A to Buffer B (Buffer A + 1 M KCl) was initiated . At the end of the gradient, Buffer B was continued for 20 min., then a linear gradient of Buffer B to Buffer A was initiated (20 mL total gradient volume). At the end of the second gradient, the column was washed with Buffer A for 20 min. The collected fractions were stored at 4°C until assayed for protein on 10% polyacrylamide, 0.1% SDS gels with 4% stacking gels and assayed for polymerase activity as described above.

Polymerase activity eluted at about 0.3 M KCl, correlating with one of the absorbing peaks of the UV absorbance trace. There was about a two-fold concentration of activity when compared to the volume of sample loaded on the column. Photography of polyacrylamide gels stained with 30% methanol, 10% acetic acid, 0.05% Coomassie Brilliant Blue R-250 and destained in 30% methanol, 10% acetic acid showed that the protein was about 50% pure. The polymerase migrated slightly below the 68kd marker, consistent with its predicted size.

The active fractions from the DEAE-cellulose column were pooled and dialyzed against KKEGD buffer (20 mM $K_2PO_4$ pH 7.4, 20 mM KCl, 10% glycerol (w/v), 1 mM DTT, 0.5 mM $Na_2$EDTA) for 4 hr. at 4°C. One mL of the dialyzed DEAE pool was diluted with 1 mL of KKEGD buffer and injected into a HITRAP Heparin column (Pharmacia) equilibrated with 20 mL of KKEGD buffer. Buffer flow was initiated at 0.25 mL/min. until UV absorbing material was detected in the eluate. The flow rate was then increased to 0.5 mL/min. and the column was washed for 10 min. with KKEGD buffer. A linear gradient from KKEGD to KKEGD, 1 M KCl was initiated (10 mL total gradient volume) and 0.5 mL fractions were collected. At the end of the gradient the KKEGD, 1 M KCl wash was continued for 10 min. and a linear gradient from KKEGD, 1 M KCl to KKEGD was initiated (5 mL total gradient volume). At the end of the gradient the column was washed with KKEGD for 10 min.. The collected fractions were stored at 4°C until assayed for protein and polymerase activity. The UV absorbance profile showed only a single peak.

Analysis on polyacrylamide gels indicated the polymerase was greater than 90% pure after heparin column chromatography. The fractions containing the highest levels of polymerase activity were pooled, concentrated and exchanged into KKEGD buffer by CENTRICON-30 ultrafiltration. The unit concentration of the purified stock was about 38,000 units/mL and the total protein concentration was about 0.6 mg/mL based on a Bradford assay. Yield was about 30 µg from one third of the DEAE active pool. The yield per gram of cells was therefore calculated to be about 70 µg, i.e., about 1 mg of protein per 14 g of cells. The estimated specific activity of this prep was 63,000 units/mg of protein.

It will be apparent from the foregoing description of the invention that several modifications are possible. First, the MBP affinity purification tag may be linked to either the N-terminus or the C-terminus of Bpa Pol I. Histidine residues (poly-histidine) added at the C-terminus or the N-terminus provide a heterologous amino acid sequence which is useful for purification of the polymerase by immobilized metal affinity chromatography (IMAC, e.g., nickel). The heterologous amino acids in the fusion protein do not adversely affect polymerase activity. Further, two heterologous sequences (e.g., two affinity purification tags) may be linked to the polymerase (one at each end), if desired, without significant adverse effects on polymerase activity. Additional alternative heterologous amino acid sequences are known in the art and may be linked to Bpa Pol I by conventional methods to produce various fusion proteins. Materials and methods for linking heterologous coding sequences such as affinity purification tags to the ends of the Bpa Pol I coding sequence and expression of the fusion proteins are well known in the art, as are methods for affinity purification of the fusion protein when the heterologous sequence is an affinity purification tag.

Further, due to degeneracy of the genetic code, different but equivalent nucleotide sequences which code for the Bpa Pol I enzyme of the invention (e.g., as shown in SEQ ID NO:4, amino acids 192-876 of SEQ ID NO:4, SEQ ID NO:6 or SEQ ID NO:8) may be isolated or prepared without the exercise of inventive skill. Such degenerate coding sequences are included within the scope of the invention. It is also within the ordinary skill in the art to clone DNA fragments encoding the Bpa Pol I of the invention into a variety of cloning vectors and to express the recombinant Bpa Pol I protein under the control of a promoter in a variety of transformed prokaryotic and eukaryotic host cells.

Extensive amino acid sequence analyses of prior art polymerase I enzymes have revealed highly conserved motifs. These motifs are known to correspond to regions of the protein required for the various activities. See, for example, L. Blanco, et al. 1991. Gene 100:27-38; M. Delarue, et al. 1990. Prot. Eng. 3:461-467; P. Gutman and K. Minton. 1993. Nucl. Acids Res. 21:4406-4407. By comparing the amino acid sequence of the Bpa Pol I of the invention to the teachings of these publications and others, it will be possible to identify the corresponding conserved motifs in the inventive polymerase. Amino acid substitutions in the regions of the protein outside the conserved motifs, particularly conservative amino acid substitutions, would be expected to have little if any effect on the biological activity of the polymerase. One skilled in the art would therefore expect that conservative (and certain non-conservative) amino acid substitutions in regions outside the conserved motifs would result in a Bpa Pol I variant which is substantially equivalent to the enzyme having the specific amino acid sequences herein disclosed. Such minor sequence variations are exemplified, but not limited to, the variant C-terminal sequences of SEQ ID NOs:6 and 8, where the amino acid sequence was altered in making the linkage to the histidine affinity purification tag. These variant Bpa Pol I polymerases and the nucleic acid sequences which encode them are considered to be substantial equivalents of the Bpa Pol I of the invention and are intended to be included within its scope.

Monoclonal and polyclonal antibodies which recognize and bind to Bpa Pol I may be prepared using the Bpa Pol I of the invention. Polyclonal antibodies are generally produced by immunizing animals with an enriched or purified preparation of Bpa Pol I according to conventional protocols. The preferred antigen for immunization is a preparation of isolated or purified Bpa Pol I, but crude extracts may also be used. Isolated recombinant Bpa Pol I is the preferred antigen for immunization. The serum of animals thus immunized contains the polyclonal anti-Bpa Pol I antibodies, and the immune serum is often used directly in immunoassays. In this case, the anti-Bpa Pol I polyclonal antibodies are isolated in serum by separating the serum from red blood cells and other cellular components of blood. Monoclonal antibodies which recognize the Bpa Pol I of the invention may be prepared using the methods of Kohler and Milstein (1975. *Nature* 256:495). Mice may be immunized with the Bpa Pol I antigen preparation, the spleen cells fused and the resulting hybridomas screened in enzyme-linked immunosorbent assays (ELISAs) or immunoblots for reactivity with the immunogen. Hybridomas secreting antibodies of interest are typically subcloned. The monoclonal antibody may be then be produced by culturing the hybridoma or in ascites in pristane-primed Balb/C mice. It is then typically isolated from the culture medium or ascites by chromatography on Protein A-Sepharose (Sigma Chemical Co., St. Louis, MO). Alternatively, Bpa Pol I may be coupled to a solid phase and used to purify polyclonal or monoclonal antibodies by affinity chromatography. Using these methods, a variety of hybridomas which produce anti-Bpa Pol I monoclonal antibodies may be identified. Monoclonal antibodies are preferred for immunoassays due to their improved specificity and affinity for the antigen to be detected. Anti-Bpa Pol I monoclonal antibodies which recognize different epitopes on the protein may also be useful in studies to evaluate protein structure and function. Anti-Bpa Pol I antibodies as described above, preferably monoclonal antibodies, may also be coupled to Protein A-Sepharose as is known in the art for use in affinity purification of naturally-occurring or recombinant Bpa Pol I.

## Claims

1. An isolated nucleic acid sequence selected from the group consisting of nucleic acid sequences encoding SEQ ID NO:4, nucleic acid sequences encoding amino acids 192-876 of SEQ ID NO:4, SEQ ID NO:3, nucleotides 574-2628 of SEQ ID NO:3, SEQ ID NO:5, nucleotides 1-3237 of SEQ ID NO:5, nucleic acid sequences encoding amino acids 1-1078 of SEQ ID NO:6, SEQ ID NO:7, nucleotides 1-3810 of SEQ ID NO:7, and nucleic acid sequences encoding amino acids 1-1270 of SEQ ID NO:8.

2. A recombinant vector comprising a nucleic acid sequence according to Claim 1.

3. The recombinant vector of Claim 2 wherein the nucleic acid sequence is linked to a nucleic acid sequence encoding a heterologous amino acid sequence.

4. A host cell transformed with the recombinant vector of Claim 2.

5. A method for producing a recombinant DNA polymerase comprising culturing the transformed host cell of Claim 4 in a culture medium under conditions whereby the recombinant DNA polymerase is expressed by the transformed host cell.

6. The method of Claim 5 further comprising purifying the expressed recombinant DNA polymerase from a cell lysate or the culture medium.

7. An isolated DNA polymerase selected from the group consisting of SEQ ID NO:4, amino acids 192-876 of SEQ ID NO:4, fusion proteins comprising SEQ ID NO:4, and fusion proteins comprising amino acids 192-876 of SEQ ID NO:4.

8. The DNA polymerase of Claim 7 wherein the fusion protein is SEQ ID NO:6 or SEQ ID NO:8.

9. A method of making a recombinant vector for expression of a DNA polymerase in a transformed host cell comprising cloning a nucleic acid sequence selected from the group consisting of SEQ ID NO:3, nucleotides 574-2628 of SEQ ID NO:3, nucleic acid sequences encoding SEQ ID NO:4 and nucleic acid sequences encoding amino acids 192-876 of SEQ ID NO:4 into an expression vector under control of a promoter such that the polymerase is expressible in the transformed host cell.

10. The method of Claim 9 further comprising linking the nucleic acid sequence in the vector to a nucleic acid sequence encoding a heterologous amino acid sequence such that expression of the polymerase produces a fusion protein.

11. An isolated anti-Bpa Pol I antibody, said Bpa Pol I having the amino acid sequence of any of the polymerases of claims 7.

12. A hybridoma which produces an anti-Bpa Pol I antibody, said Bpa Pol I having the amino acid sequence of any of the polymerases of claims 7.

## Patentansprüche

1. Isolierte Nucleinsäuresequenz, ausgewählt aus der Gruppe bestehend aus Nucleinsäuresequenzen, welche SEQ ID NO: 4 codieren, Nucleinsäuresequenzen, welche die Aminosäuren 192-876 von SEQ ID NO: 4 codieren, SEQ ID NO: 3, den Nucleotiden 574-2628 von SEQ ID NO: 3, SEQ ID NO: 5, den Nucleotiden 1-3237 von SEQ ID NO: 5, Nucleinsäuresequenzen, welche die Aminosäuren 1-1078 von SEQ ID NO: 6 codieren, SEQ ID NO: 7, den Nucleotiden 1-3810 von SEQ ID NO: 7 und Nucleinsäuresequenzen, welche die Aminosäuren 1-1270 von SEQ ID NO: 8 codieren.

2. Rekombinanter Vektor, der eine Nucleinsäuresequenz nach Anspruch 1 umfaßt.

3. Rekombinanter Vektor nach Anspruch 2, wobei die Nucleinsäuresequenz mit einer Nucleinsäuresequenz verknüpft ist, welche eine heterologe Aminosäuresequenz codiert.

4. Wirtszelle, die mit dem rekombinanten Vektor von Anspruch 2 transformiert ist.

5. Verfahren zur Herstellung einer rekombinanten DNA-Polymerase, das die Kultivierung der transformierten Wirtszelle von Anspruch 4 in einem Kulturmedium unter Bedingungen umfaßt, durch welche die rekombinante DNA-Polymerase von der transformierten Wirtszelle exprimiert wird.

6. Verfahren nach Anspruch 5, das außerdem die Aufreinigung der exprimierten rekombinanten DNA-Polymerase aus einem Zell-Lysat oder dem Kulturmedium umfaßt.

7. Isolierte DNA-Polymerase, ausgewählt aus der Gruppe bestehend aus SEQ ID NO: 4, den Aminosäuren 192-876 von SEQ ID NO: 4, Fusionsproteinen, welche SEQ ID NO: 4 umfassen, und Fusionsproteinen, welche die Aminosäuren 192-876 von SEQ ID NO: 4 umfassen.

8. DNA-Polymerase nach Anspruch 7, wobei das Fusionsprotein SEQ ID NO: 6 oder SEQ ID NO: 8 ist.

9. Verfahren zur Herstellung eines rekombinanten Vektors zur Expression einer DNA-Polymerase in einer transformierten Wirtszelle, welches das Klonieren einer Nucleinsäuresequenz, ausgewählt aus der Gruppe bestehend aus SEQ ID NO: 3, den Nucleotiden 574-2628 von SEQ ID NO: 3, Nucleinsäuresequenzen, welche SEQ ID NO: 4 codieren, und Nucleinsäuresequenzen, welche die Aminosäuren 192-876 von SEQ ID NO: 4 codieren, in einen Expressionsvektor unter der Kontrolle eines Promotors umfaßt, so daß die Polymerase in der transformierten Wirtszelle exprimierbar ist.

10. Verfahren nach Anspruch 9, das außerdem die Verknüpfung der Nucleinsäuresequenz in dem Vektor mit einer Nucleinsäuresequenz umfaßt, welche eine heterologe Aminosäuresequenz codiert, so daß die Expression der Polymerase ein Fusionsprotein ergibt.

11. Isolierter Anti-Bpa-Pol-I-Antikörper, wobei Bpa Pol I die Aminosäuresequenz irgendeiner der Polymerasen von Anspruch 7 aufweist.

12. Hybridom, das einen Anti-Bpa-Pol-I-Antikörper produziert, wobei Bpa Pol I die Aminosäuresequenz irgendeiner der Polymerasen von Anspruch 7 aufweist.

## Revendications

1. Séquence d'acide nucléique isolée choisie dans le groupe consistant en des séquences d'acide nucléique codant pour la Séquence identifiée par le N° 4, des séquences d'acide nucléique codant pour les acides aminés 192 - 876 de la Séquence identifiée par le N° 4, de la séquence identifiée par le N° 3, les nucléotides 574 - 2628 de la Séquence identifiée par le N° 3, de la Séquence identifiée par le N° 5, les nucléotides 1 - 3237 de la Séquence identifiée par le N° 5, les nucléotides 1 - 1078 de la Séquence identifiée par le N° 6, de la Séquence identifiée par le N° 7, les nucléotides 1 - 3810 de la Séquence identifiée par le N° 7, et les séquences d'acide nucléique codant pour les acides aminés 1 - 1270 de la Séquence identifiée par le N° 8.

2. Vecteur recombinant comprenant une séquence d'acide nucléique selon la revendication 1.

3. Vecteur recombinant selon la revendication 2, dans lequel la séquence d'acide nucléique est liée à une séquence d'acide nucléique codant pour une séquence d'acides aminés hétérologue.

4. Cellule hôte transformée par le vecteur recombinant selon la revendication 2.

5. Procédé de production d'une ADN-polymérase recombinante comprenant la mise en culture d'une cellule hôte transformée selon la revendication 4 dans un milieu de culture dans des conditions permettant l'expression de l'ADN-polymérase recombinante par la cellule hôte transformée.

6. Procédé selon la revendication 5 comprenant en outre la purification de l'ADN-polymérase recombinante exprimée à partir du lysat cellulaire ou du milieu de culture.

7. ADN-polymérase isolée choisie dans le groupe consistant en la Séquence identifiée par le N° 4, les acides aminés 192 - 876 de la Séquence identifiée par le N° 4, des protéines de fusion comprenant la Séquence identifiée par le N° 4; et des protéines de fusion comprenant les acides aminés 192 - 876 de la Séquence identifiée par le N° 4.

**8.** ADN-polymérase selon la revendication 7, dans laquelle la protéine de fusion est la Séquence identifiée par le N° 6 ou la Séquence identifiée par le N° 8.

**9.** Procédé del production d'un vecteur recombinant pour l'expression d'une ADN-polymérase dans une cellule hôte transformée comprenant le clonage d'une séquence d'acide nucléique choisie dans le groupe consistant en la Séquence identifiée par le N° 3, les nucléotides 574 - 2628 de la Séquence identifiée par le N° 3, les séquences d'acide nucléique codant pour la Séquence Identifiée par le N° 4 et les séquences d'acide nucléique codant pour les acides aminés 192 - 876 de la Séquence identifiée par le N° 4 dans un vecteur d'expression sous la dépendance d'un promoteur de nature à permettre l'expression de la polymérase dans la cellule hôte transformée.

**10.** Procédé selon la revendication 9 comprenant en outre la liaison de la séquence d'acide nucléique insérée dans le vecteur à une séquence d'acide nucléique codant pour une séquence d'acides aminés hétérologue de telle sorte que l'expression de la polymérase produise une protéine de fusion.

**11.** Anticorps anti-Bpa Pol I isolé, ladite Bpa Pol I ayant la séquence d'acides aminés de l'une quelconque des polymérases selon la revendication 7.

**12.** Hybridome qui produit un anticorps anti-Bpa Pol I, ladite Bpa Pol I ayant la séquence d'acides aminés de l'une quelconque des polymérases selon la revendication 7.

SEQUENCE LISTING:

(1) GENERAL INFORMATION:

(i) APPLICANT: BECTON, DICKINSON AND COMPANY

(A) NAME: BECTON, DICKINSON AND COMPANY
(B) STREET: 1 Becton Drive
(C) CITY: Franklin Lakes
(D) STATE: New Jersey
(E) COUNTRY: USA
(F) ZIP: 07417

(ii) TITLE OF INVENTION: THERMOSTABLE DNA POLYMERASE FROM BACILLUS PALLIDUS

(iii) NUMBER OF SEQUENCES: 8

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(v) CURRENT APPLICATION DATA:

(A) APPLICATION NUMBER:
(B) FILING DATE:
(C) CLASSIFICATION:

(2) INFORMATION FOR SEQ ID NO:1:

(i) SEQUENCE CHARACTERISTICS:

(A) LENGTH: 39 base pairs
(B) TYPE: nucleic acid
(C) STRANDEDNESS: single
(D) TOPOLOGY: linear

(xi) SEQUENCE DESCRIPTION: SEQ ID NO:1:

TGCCAATCTA GAAGGCGTGC CSGGCRTCGG KRAAAARAC 39

(2) INFORMATION FOR SEQ ID NO:2:

(i) SEQUENCE CHARACTERISTICS:

(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:2:

CACCAAGGAT CCSYTTTTAT TTSGCGTCRT ACCAYGT 37

(2) INFORMATION FOR SEQ ID NO:3:

(i) SEQUENCE CHARACTERISTICS:

(A) LENGTH: 2631 base pairs
(B) TYPE: nucleic acid

(C) STRANDEDNESS: double
(D) TOPOLOGY: linear

(ix) FEATURE:

(A) NAME/KEY: CDS
(B) LOCATION: 1..2628

(xi) SEQUENCE DESCRIPTION: SEQ ID NO:3:

```
GTG ACA AAG AAG CTA GTT TTA ATT GAT GGA AAC AGT ATT GCT TAC AGA      48
Val Thr Lys Lys Leu Val Leu Ile Asp Gly Asn Ser Ile Ala Tyr Arg
  1               5                  10                  15

GCG TTT TTC GCT TTG CCG CTT TTA AAT AAT GAT AAG GGG ATT TAT ACG      96
Ala Phe Phe Ala Leu Pro Leu Leu Asn Asn Asp Lys Gly Ile Tyr Thr
             20                  25                  30

AAT GCA ATT TAC GGC TTT ACA AAT ATG CTG TTA AAA GTA CTG GAG GAA     144
Asn Ala Ile Tyr Gly Phe Thr Asn Met Leu Leu Lys Val Leu Glu Glu
         35                  40                  45

GAA AAA CCG ACA CAT ATT CTT GTT GCA TTT GAT GCA GGG AAA ACA ACG     192
Glu Lys Pro Thr His Ile Leu Val Ala Phe Asp Ala Gly Lys Thr Thr
     50                  55                  60

TTC CGG CAT AAA ACT TTT AAA GAA TAT AAA GGA ACT CGG CAA AAA ACC     240
Phe Arg His Lys Thr Phe Lys Glu Tyr Lys Gly Thr Arg Gln Lys Thr
 65                  70                  75                  80

CCG CCT GAA TTG TCG GAG CAG CTA CCA TTT ATA CGG GAT TTG CTT GAT     288
Pro Pro Glu Leu Ser Glu Gln Leu Pro Phe Ile Arg Asp Leu Leu Asp
                 85                  90                  95

GCC TAC CAA ATT ACA ACA TAT GAA CTC GAA AAT TAT GAG GCT GAT GAT     336
Ala Tyr Gln Ile Thr Thr Tyr Glu Leu Glu Asn Tyr Glu Ala Asp Asp
            100                 105                 110

ATT ATT GGA ACA GTT GCG AGA CAA GCT GAG AAG CAA GAT TTT GAA GTG     384
Ile Ile Gly Thr Val Ala Arg Gln Ala Glu Lys Gln Asp Phe Glu Val
        115                 120                 125

AAA ATT ATT TCC GGA GAT AAG GAT TTA ACA CAG CTG GCA ACT GAA AAA     432
Lys Ile Ile Ser Gly Asp Lys Asp Leu Thr Gln Leu Ala Thr Glu Lys
    130                 135                 140

ACG ACC GTT TCC ATC ACG AAA AAA GGA ATT ACA GAT GTT GAA CCG CAC     480
Thr Thr Val Ser Ile Thr Lys Lys Gly Ile Thr Asp Val Glu Pro His
145                 150                 155                 160

ACG CCT GAA TCG ATT CAA GAG AAG TAT GGG CTA AGC CCG GCA CAA ATT     528
Thr Pro Glu Ser Ile Gln Glu Lys Tyr Gly Leu Ser Pro Ala Gln Ile
                165                 170                 175

ATT GAT TTG AAA GGA TTG ATG GGC GAT CAA TCA GAT AAT ATC CCA GGT     576
```

```
Ile Asp Leu Lys Gly Leu Met Gly Asp Gln Ser Asp Asn Ile Pro Gly
            180                 185                 190

GTG CCC GGC GTT GGA GAG AAA ACC GCG ATT AAA TTG CTG AAA CAG TTT      624
Val Pro Gly Val Gly Glu Lys Thr Ala Ile Lys Leu Leu Lys Gln Phe
        195                 200                 205

GAG ACA GTC GAA AAT ATT TTA AAT TCG ATT GAA GAA GTA AAT GGA AAA      672
Glu Thr Val Glu Asn Ile Leu Asn Ser Ile Glu Glu Val Asn Gly Lys
    210                 215                 220

AAA TTG AAG GAA AAC TTA CAA AAC TAT AAA GAG CAA GCA TTA ATG AGC      720
Lys Leu Lys Glu Asn Leu Gln Asn Tyr Lys Glu Gln Ala Leu Met Ser
225                 230                 235                 240

AAA CAG CTT GCG ACA ATT CAT TGT GAA GCT CCT GTC GAA ATA AAA ATT      768
Lys Gln Leu Ala Thr Ile His Cys Glu Ala Pro Val Glu Ile Lys Ile
                245                 250                 255

CAA GAC CTT GAG TAT AAA GGC TAT GAC AAA GAA AAA GTA GTG AAA ATT      816
Gln Asp Leu Glu Tyr Lys Gly Tyr Asp Lys Glu Lys Val Val Lys Ile
            260                 265                 270

TTT AAG GAA CTA GGC TTC CAA TCG CTC CTA GAC AAA ATG GGA GAG CAT      864
Phe Lys Glu Leu Gly Phe Gln Ser Leu Leu Asp Lys Met Gly Glu His
        275                 280                 285

GAG AAT GAA GAA GCG GAT GAA ATG CCG ACG ATT AAG TTC GAA AAA GTT      912
Glu Asn Glu Glu Ala Asp Glu Met Pro Thr Ile Lys Phe Glu Lys Val
    290                 295                 300

GAA AAG CTG TCA GAC AAG GTT TTA TCA GAG AAG GCA GCT CTT TTA GTG      960
Glu Lys Leu Ser Asp Lys Val Leu Ser Glu Lys Ala Ala Leu Leu Val
305                 310                 315                 320

GAA ATC ATT GAT GAA AAT TAT CAT ACT GGA GAA ATC ATC GGG TTT TCT     1008
Glu Ile Ile Asp Glu Asn Tyr His Thr Gly Glu Ile Ile Gly Phe Ser
                325                 330                 335

ATC GCA AAC GAA AAT GGA TGT TTT TAT ATT CCA GCC GAA ATT GCG CTA     1056
Ile Ala Asn Glu Asn Gly Cys Phe Tyr Ile Pro Ala Glu Ile Ala Leu
            340                 345                 350

CAT TCA AAA GAG TTC ATA GAA TGG GTG AAG GAT GAA ACA AAG CGG AAA     1104
His Ser Lys Glu Phe Ile Glu Trp Val Lys Asp Glu Thr Lys Arg Lys
        355                 360                 365

GTG GTG TAT GAT GCG AAA AAA TCA ATT GTG GCG CTG CGC TGG CGA AAC     1152
Val Val Tyr Asp Ala Lys Lys Ser Ile Val Ala Leu Arg Trp Arg Asn
    370                 375                 380

ATT GAT TTA GCA GGT ATT GAG TTT GAT GTT CTC ATT GCC TCA TAC ATT     1200
Ile Asp Leu Ala Gly Ile Glu Phe Asp Val Leu Ile Ala Ser Tyr Ile
385                 390                 395                 400

TTA AAT CCG TCT GAA TCG ATT GAC GAC ATA GCC GAG CTT GCC AAG ACA     1248
Leu Asn Pro Ser Glu Ser Ile Asp Asp Ile Ala Glu Leu Ala Lys Thr
                405                 410                 415

AAA AAT AAA CAT TTA GTT CAA AAG GAT GAA GTG ATT TAC GGA AAA GGC     1296
Lys Asn Lys His Leu Val Gln Lys Asp Glu Val Ile Tyr Gly Lys Gly
            420                 425                 430
```

```
GCT AAA CGT CAT ATC CCT GAT GAA GAC ATT TTA GGC GAA CAT CTT GCC    1344
Ala Lys Arg His Ile Pro Asp Glu Asp Ile Leu Gly Glu His Leu Ala
        435                 440                 445

AGA AAA GCG TTA GCC ATT TAT GAG CTG GAA GAA TTA TTA ATA CAA GAA    1392
Arg Lys Ala Leu Ala Ile Tyr Glu Leu Glu Glu Leu Leu Ile Gln Glu
    450                 455                 460

TTA GAA GAA AAT GAA CAA TTT CAT TTA TTC AGC GAA TTG GAG CTT CCG    1440
Leu Glu Glu Asn Glu Gln Phe His Leu Phe Ser Glu Leu Glu Leu Pro
465                 470                 475                 480

CTG TCA GCC ATT TTA TCT GAC ATG GAA ACA ACA GGA GTA AAG ATA GAC    1488
Leu Ser Ala Ile Leu Ser Asp Met Glu Thr Thr Gly Val Lys Ile Asp
                485                 490                 495

GTC AAC CGT CTG AAA GAA ATG GGA AAA GAG CTT GAT GAA CAG CTG AAG    1536
Val Asn Arg Leu Lys Glu Met Gly Lys Glu Leu Asp Glu Gln Leu Lys
            500                 505                 510

CAA TTA GAA AAG GAT ATT CAT CGT CTA GCT GGA GTG TCA TTT AAC ATT    1584
Gln Leu Glu Lys Asp Ile His Arg Leu Ala Gly Val Ser Phe Asn Ile
        515                 520                 525

AAT TCT CCG AAG CAG CTT GGG CCG ATT TTA TTT GAA AAG CTC AAT CTA    1632
Asn Ser Pro Lys Gln Leu Gly Pro Ile Leu Phe Glu Lys Leu Asn Leu
    530                 535                 540

CCG GTT TTG AAA AAG ACC AAA ACG GGG TAT TCG ACC TCT GCG GAC GTT    1680
Pro Val Leu Lys Lys Thr Lys Thr Gly Tyr Ser Thr Ser Ala Asp Val
545                 550                 555                 560

TTA GAA AAA TTG AGA GGA CAG CAC GAA ATT GTG GAG AAA ATT TTG CAT    1728
Leu Glu Lys Leu Arg Gly Gln His Glu Ile Val Glu Lys Ile Leu His
                565                 570                 575

TAC CGG CAG CTC GGA AAG CTT CAA TCG ACG TAT ATT GAA GGG CTG CTG    1776
Tyr Arg Gln Leu Gly Lys Leu Gln Ser Thr Tyr Ile Glu Gly Leu Leu
            580                 585                 590

AAG GTT GTC CAT CGT GAT ACG CAT AAA ATC CAC ACC CGA TTT AAT CAA    1824
Lys Val Val His Arg Asp Thr His Lys Ile His Thr Arg Phe Asn Gln
        595                 600                 605

GCA TTA ACG CAA ACC GGA AGA TTA AGC TCC ACA GAC CCG AAT TTG CAA    1872
Ala Leu Thr Gln Thr Gly Arg Leu Ser Ser Thr Asp Pro Asn Leu Gln
    610                 615                 620

AAC ATT CCG ATT CGC CTT GAG GAA GGC CGC AAA ATT CGT CAA GCA TTT    1920
Asn Ile Pro Ile Arg Leu Glu Glu Gly Arg Lys Ile Arg Gln Ala Phe
625                 630                 635                 640

ATC CCT TCT GAA AAA GAT TGG GTC ATT TTT GCA GCG GAC TAT TCC CAG    1968
Ile Pro Ser Glu Lys Asp Trp Val Ile Phe Ala Ala Asp Tyr Ser Gln
                645                 650                 655

ATT GAA CTG CGA GTG CTT GCG CAT ATA TCT GGA GAT GAA AAA TTG ATT    2016
Ile Glu Leu Arg Val Leu Ala His Ile Ser Gly Asp Glu Lys Leu Ile
            660                 665                 670

GAA GCG TTT AAA CAA GAT CTT GAT ATT CAT ACA AAA ACG GCG ATC GAT    2064
Glu Ala Phe Lys Gln Asp Leu Asp Ile His Thr Lys Thr Ala Ile Asp
        675                 680                 685
```

```
GTG TTC CAT GTC GAA GAA GAT AAA GTG ACC TCC AAC ATG AGA AGA CAG        2112
Val Phe His Val Glu Glu Asp Lys Val Thr Ser Asn Met Arg Arg Gln
    690                 695                 700

GCA AAA GCA GTT AAT TTC GGG ATT GTT TAC GGA ATC AGC GAT TAC GGA        2160
Ala Lys Ala Val Asn Phe Gly Ile Val Tyr Gly Ile Ser Asp Tyr Gly
705                 710                 715                 720

TTG TCG CAA AAC TTA GGA ATT ACC CGA AAA GAA GCT GGT GAA TTT ATT        2208
Leu Ser Gln Asn Leu Gly Ile Thr Arg Lys Glu Ala Gly Glu Phe Ile
                725                 730                 735

AAA AAA TAT TTT GAA ATT TAT AAA GGC GTT AAA GAA TAT ATG GAT GGC        2256
Lys Lys Tyr Phe Glu Ile Tyr Lys Gly Val Lys Glu Tyr Met Asp Gly
            740                 745                 750

ATA ATC CAA GAG GCG AAG CAA AAA GGC TAT GTA ACG ACA CTA ATG CAG        2304
Ile Ile Gln Glu Ala Lys Gln Lys Gly Tyr Val Thr Thr Leu Met Gln
        755                 760                 765

CGT CGG AGA TAT ATT CCG GAA ATT ACG AGC AGA AAT TTC AAT ATC AGA        2352
Arg Arg Arg Tyr Ile Pro Glu Ile Thr Ser Arg Asn Phe Asn Ile Arg
    770                 775                 780

AGC TTC GCT GAG CGA ACA GCC ATG AAT ACT CCG ATT CAA GGA AGT GCA        2400
Ser Phe Ala Glu Arg Thr Ala Met Asn Thr Pro Ile Gln Gly Ser Ala
785                 790                 795                 800

GCG GAT ATT ATC AAA AAA GCG ATG ATC GAT ATG GCG CAA GAA ATT GAA        2448
Ala Asp Ile Ile Lys Lys Ala Met Ile Asp Met Ala Gln Glu Ile Glu
                805                 810                 815

AAA CGA AAT TTG CAA ACG AGG CTG CTG CTT CAA GTT CAT GAC GAA TTG        2496
Lys Arg Asn Leu Gln Thr Arg Leu Leu Leu Gln Val His Asp Glu Leu
            820                 825                 830

GTG TTT GAA GCG CCA AAG GAT GAA ATT GAA ATT TTA GAA AAG CTT GTT        2544
Val Phe Glu Ala Pro Lys Asp Glu Ile Glu Ile Leu Glu Lys Leu Val
        835                 840                 845

CCG GAA GTA ATG GAA AAT GCC ATT CAG CTA AAA GTA CCG TTA AAG GTT        2592
Pro Glu Val Met Glu Asn Ala Ile Gln Leu Lys Val Pro Leu Lys Val
    850                 855                 860

GAT TAT TCT TAC GGT TCT ACG TGG TAT GAA GCG AAA TAA                    2631
Asp Tyr Ser Tyr Gly Ser Thr Trp Tyr Glu Ala Lys
865                 870                 875
```

(2) INFORMATION FOR SEQ ID NO:4:

(i) SEQUENCE CHARACTERISTICS:

(A) LENGTH: 876 amino acids
(B) TYPE: amino acid
(D) TOPOLOGY: linear

(ii) MOLECULE TYPE: protein

(xi) SEQUENCE DESCRIPTION: SEQ ID NO:4:

```
Val Thr Lys Lys Leu Val Leu Ile Asp Gly Asn Ser Ile Ala Tyr Arg
 1               5                  10                  15
```

Ala Phe Phe Ala Leu Pro Leu Leu Asn Asn Asp Lys Gly Ile Tyr Thr
20 25 30

Asn Ala Ile Tyr Gly Phe Thr Asn Met Leu Leu Lys Val Leu Glu Glu
35 40 45

Glu Lys Pro Thr His Ile Leu Val Ala Phe Asp Ala Gly Lys Thr Thr
50 55 60

Phe Arg His Lys Thr Phe Lys Glu Tyr Lys Gly Thr Arg Gln Lys Thr
65 70 75 80

Pro Pro Glu Leu Ser Glu Gln Leu Pro Phe Ile Arg Asp Leu Leu Asp
85 90 95

Ala Tyr Gln Ile Thr Thr Tyr Glu Leu Glu Asn Tyr Glu Ala Asp Asp
100 105 110

Ile Ile Gly Thr Val Ala Arg Gln Ala Glu Lys Gln Asp Phe Glu Val
115 120 125

Lys Ile Ile Ser Gly Asp Lys Asp Leu Thr Gln Leu Ala Thr Glu Lys
130 135 140

Thr Thr Val Ser Ile Thr Lys Lys Gly Ile Thr Asp Val Glu Pro His
145 150 155 160

Thr Pro Glu Ser Ile Gln Glu Lys Tyr Gly Leu Ser Pro Ala Gln Ile
165 170 175

Ile Asp Leu Lys Gly Leu Met Gly Asp Gln Ser Asp Asn Ile Pro Gly
180 185 190

Val Pro Gly Val Gly Glu Lys Thr Ala Ile Lys Leu Leu Lys Gln Phe
195 200 205

Glu Thr Val Glu Asn Ile Leu Asn Ser Ile Glu Glu Val Asn Gly Lys
210 215 220

Lys Leu Lys Glu Asn Leu Gln Asn Tyr Lys Glu Gln Ala Leu Met Ser
225 230 235 240

Lys Gln Leu Ala Thr Ile His Cys Glu Ala Pro Val Glu Ile Lys Ile
245 250 255

Gln Asp Leu Glu Tyr Lys Gly Tyr Asp Lys Glu Lys Val Val Lys Ile
260 265 270

Phe Lys Glu Leu Gly Phe Gln Ser Leu Leu Asp Lys Met Gly Glu His
275 280 285

Glu Asn Glu Glu Ala Asp Glu Met Pro Thr Ile Lys Phe Glu Lys Val
290 295 300

Glu Lys Leu Ser Asp Lys Val Leu Ser Glu Lys Ala Ala Leu Leu Val
305 310 315 320

Glu Ile Ile Asp Glu Asn Tyr His Thr Gly Glu Ile Ile Gly Phe Ser
325 330 335

Ile Ala Asn Glu Asn Gly Cys Phe Tyr Ile Pro Ala Glu Ile Ala Leu
340 345 350

His Ser Lys Glu Phe Ile Glu Trp Val Lys Asp Glu Thr Lys Arg Lys
355 360 365

Val Val Tyr Asp Ala Lys Lys Ser Ile Val Ala Leu Arg Trp Arg Asn
370 375 380

Ile Asp Leu Ala Gly Ile Glu Phe Asp Val Leu Ile Ala Ser Tyr Ile
385 390 395 400

Leu Asn Pro Ser Glu Ser Ile Asp Asp Ile Ala Glu Leu Ala Lys Thr
405 410 415

Lys Asn Lys His Leu Val Gln Lys Asp Glu Val Ile Tyr Gly Lys Gly
420 425 430

Ala Lys Arg His Ile Pro Asp Glu Asp Ile Leu Gly Glu His Leu Ala
435 440 445

Arg Lys Ala Leu Ala Ile Tyr Glu Leu Glu Glu Leu Leu Ile Gln Glu
450 455 460

Leu Glu Glu Asn Glu Gln Phe His Leu Phe Ser Glu Leu Glu Leu Pro
465 470 475 480

Leu Ser Ala Ile Leu Ser Asp Met Glu Thr Thr Gly Val Lys Ile Asp
485 490 495

Val Asn Arg Leu Lys Glu Met Gly Lys Glu Leu Asp Glu Gln Leu Lys
500 505 510

Gln Leu Glu Lys Asp Ile His Arg Leu Ala Gly Val Ser Phe Asn Ile
515 520 525

Asn Ser Pro Lys Gln Leu Gly Pro Ile Leu Phe Glu Lys Leu Asn Leu
530 535 540

Pro Val Leu Lys Lys Thr Lys Thr Gly Tyr Ser Thr Ser Ala Asp Val
545 550 555 560

Leu Glu Lys Leu Arg Gly Gln His Glu Ile Val Glu Lys Ile Leu His
565 570 575

Tyr Arg Gln Leu Gly Lys Leu Gln Ser Thr Tyr Ile Glu Gly Leu Leu
580 585 590

Lys Val Val His Arg Asp Thr His Lys Ile His Thr Arg Phe Asn Gln
595 600 605

Ala Leu Thr Gln Thr Gly Arg Leu Ser Ser Thr Asp Pro Asn Leu Gln
610 615 620

Asn Ile Pro Ile Arg Leu Glu Glu Gly Arg Lys Ile Arg Gln Ala Phe
625 630 635 640

Ile Pro Ser Glu Lys Asp Trp Val Ile Phe Ala Ala Asp Tyr Ser Gln
645 650 655

Ile Glu Leu Arg Val Leu Ala His Ile Ser Gly Asp Glu Lys Leu Ile
660 665 670

Glu Ala Phe Lys Gln Asp Leu Asp Ile His Thr Lys Thr Ala Ile Asp
675 680 685

```
Val Phe His Val Glu Glu Asp Lys Val Thr Ser Asn Met Arg Arg Gln
    690                 695                 700

Ala Lys Ala Val Asn Phe Gly Ile Val Tyr Gly Ile Ser Asp Tyr Gly
705                 710                 715                 720

Leu Ser Gln Asn Leu Gly Ile Thr Arg Lys Glu Ala Gly Glu Phe Ile
                725                 730                 735

Lys Lys Tyr Phe Glu Ile Tyr Lys Gly Val Lys Glu Tyr Met Asp Gly
            740                 745                 750

Ile Ile Gln Glu Ala Lys Gln Lys Gly Tyr Val Thr Thr Leu Met Gln
        755                 760                 765

Arg Arg Arg Tyr Ile Pro Glu Ile Thr Ser Arg Asn Phe Asn Ile Arg
    770                 775                 780

Ser Phe Ala Glu Arg Thr Ala Met Asn Thr Pro Ile Gln Gly Ser Ala
785                 790                 795                 800

Ala Asp Ile Ile Lys Lys Ala Met Ile Asp Met Ala Gln Glu Ile Glu
                805                 810                 815

Lys Arg Asn Leu Gln Thr Arg Leu Leu Leu Gln Val His Asp Glu Leu
            820                 825                 830

Val Phe Glu Ala Pro Lys Asp Glu Ile Glu Ile Leu Glu Lys Leu Val
        835                 840                 845

Pro Glu Val Met Glu Asn Ala Ile Gln Leu Lys Val Pro Leu Lys Val
    850                 855                 860

Asp Tyr Ser Tyr Gly Ser Thr Trp Tyr Glu Ala Lys
865                 870                 875
```

(2) INFORMATION FOR SEQ ID NO:5

(i) SEQUENCE CHARACTERISTICS;

(A) LENGTH: 3255 base pairs
(B) TYPE: nucleic acid
(C) STRANDEDNESS: double
(D) TOPOLOGY: linear

(ix) FEATURE:

(A) NAME/KEY: CDS
(B) LOCATION: 1..3252

(xi) SEQUENCE DESCRIPTION: SEQ ID NO:5:

```
ATG AAA ATC GAA GAA GGT AAA CTG GTA ATC TGG ATT AAC GGC GAT AAA      48
Met Lys Ile Glu Glu Gly Lys Leu Val Ile Trp Ile Asn Gly Asp Lys
            880                 885                 890

GGC TAT AAC GGT CTC GCT GAA GTC GGT AAG AAA TTC GAG AAA GAT ACC      96
Gly Tyr Asn Gly Leu Ala Glu Val Gly Lys Lys Phe Glu Lys Asp Thr
        895                 900                 905

GGA ATT AAA GTC ACC GTT GAG CAT CCG GAT AAA CTG GAA GAG AAA TTC     144
```

```
Gly Ile Lys Val Thr Val Glu His Pro Asp Lys Leu Glu Glu Lys Phe
    910                 915                 920

CCA CAG GTT GCG GCA ACT GGC GAT GGC CCT GAC ATT ATC TTC TGG GCA        192
Pro Gln Val Ala Ala Thr Gly Asp Gly Pro Asp Ile Ile Phe Trp Ala
925                 930                 935                 940

CAC GAC CGC TTT GGT GGC TAC GCT CAA TCT GGC CTG TTG GCT GAA ATC        240
His Asp Arg Phe Gly Gly Tyr Ala Gln Ser Gly Leu Leu Ala Glu Ile
                945                 950                 955

ACC CCG GAC AAA GCG TTC CAG GAC AAG CTG TAT CCG TTT ACC TGG GAT        288
Thr Pro Asp Lys Ala Phe Gln Asp Lys Leu Tyr Pro Phe Thr Trp Asp
            960                 965                 970

GCC GTA CGT TAC AAC GGC AAG CTG ATT GCT TAC CCG ATC GCT GTT GAA        336
Ala Val Arg Tyr Asn Gly Lys Leu Ile Ala Tyr Pro Ile Ala Val Glu
        975                 980                 985

GCG TTA TCG CTG ATT TAT AAC AAA GAT CTG CTG CCG AAC CCG CCA AAA        384
Ala Leu Ser Leu Ile Tyr Asn Lys Asp Leu Leu Pro Asn Pro Pro Lys
    990                 995                 1000

ACC TGG GAA GAG ATC CCG GCG CTG GAT AAA GAA CTG AAA GCG AAA GGT        432
Thr Trp Glu Glu Ile Pro Ala Leu Asp Lys Glu Leu Lys Ala Lys Gly
1005                1010                1015                1020

AAG AGC GCG CTG ATG TTC AAC CTG CAA GAA CCG TAC TTC ACC TGG CCG        480
Lys Ser Ala Leu Met Phe Asn Leu Gln Glu Pro Tyr Phe Thr Trp Pro
                1025                1030                1035

CTG ATT GCT GCT GAC GGG GGT TAT GCG TTC AAG TAT GAA AAC GGC AAG        528
Leu Ile Ala Ala Asp Gly Gly Tyr Ala Phe Lys Tyr Glu Asn Gly Lys
            1040                1045                1050

TAC GAC ATT AAA GAC GTG GGC GTG GAT AAC GCT GGC GCG AAA GCG GGT        576
Tyr Asp Ile Lys Asp Val Gly Val Asp Asn Ala Gly Ala Lys Ala Gly
        1055                1060                1065

CTG ACC TTC CTG GTT GAC CTG ATT AAA AAC AAA CAC ATG AAT GCA GAC        624
Leu Thr Phe Leu Val Asp Leu Ile Lys Asn Lys His Met Asn Ala Asp
    1070                1075                1080

ACC GAT TAC TCC ATC GCA GAA GCT GCC TTT AAT AAA GGC GAA ACA GCG        672
Thr Asp Tyr Ser Ile Ala Glu Ala Ala Phe Asn Lys Gly Glu Thr Ala
1085                1090                1095                1100

ATG ACC ATC AAC GGC CCG TGG GCA TGG TCC AAC ATC GAC ACC AGC AAA        720
Met Thr Ile Asn Gly Pro Trp Ala Trp Ser Asn Ile Asp Thr Ser Lys
                1105                1110                1115

GTG AAT TAT GGT GTA ACG GTA CTG CCG ACC TTC AAG GGT CAA CCA TCC        768
Val Asn Tyr Gly Val Thr Val Leu Pro Thr Phe Lys Gly Gln Pro Ser
            1120                1125                1130

AAA CCG TTC GTT GGC GTG CTG AGC GCA GGT ATT AAC GCC GCC AGT CCG        816
Lys Pro Phe Val Gly Val Leu Ser Ala Gly Ile Asn Ala Ala Ser Pro
        1135                1140                1145

AAC AAA GAG CTG GCA AAA GAG TTC CTC GAA AAC TAT CTG CTG ACT GAT        864
Asn Lys Glu Leu Ala Lys Glu Phe Leu Glu Asn Tyr Leu Leu Thr Asp
    1150                1155                1160
```

GAA GGT CTG GAA GCG GTT AAT AAA GAC AAA CCG CTG GGT GCC GTA GCG 912
Glu Gly Leu Glu Ala Val Asn Lys Asp Lys Pro Leu Gly Ala Val Ala
1165 1170 1175 1180

CTG AAG TCT TAC GAG GAA GAG TTG GCG AAA GAT CCA CGT ATT GCC GCC 960
Leu Lys Ser Tyr Glu Glu Glu Leu Ala Lys Asp Pro Arg Ile Ala Ala
1185 1190 1195

ACC ATG GAA AAC GCC CAG AAA GGT GAA ATC ATG CCG AAC ATC CCG CAG 1008
Thr Met Glu Asn Ala Gln Lys Gly Glu Ile Met Pro Asn Ile Pro Gln
1200 1205 1210

ATG TCC GCT TTC TGG TAT GCC GTG CGT ACT GCG GTG ATC AAC GCC GCC 1056
Met Ser Ala Phe Trp Tyr Ala Val Arg Thr Ala Val Ile Asn Ala Ala
1215 1220 1225

AGC GGT CGT CAG ACT GTC GAT GAA GCC CTG AAA GAC GCG CAG ACT AAT 1104
Ser Gly Arg Gln Thr Val Asp Glu Ala Leu Lys Asp Ala Gln Thr Asn
1230 1235 1240

TCG AGC TCG AAC AAC AAC AAC AAT AAC AAT AAC AAC AAC CTC GGG ATC 1152
Ser Ser Ser Asn Asn Asn Asn Asn Asn Asn Asn Asn Asn Leu Gly Ile
1245 1250 1255 1260

GAG GGA AGG ATT TCA GAA TTC GGT GTG CCC GGC GTT GGA GAG AAA ACC 1200
Glu Gly Arg Ile Ser Glu Phe Gly Val Pro Gly Val Gly Glu Lys Thr
1265 1270 1275

GCG ATT AAA TTG CTG AAA CAG TTT GAG ACA GTC GAA AAT ATT TTA AAT 1248
Ala Ile Lys Leu Leu Lys Gln Phe Glu Thr Val Glu Asn Ile Leu Asn
1280 1285 1290

TCG ATT GAA GAA GTA AAT GGA AAA AAA TTG AAG GAA AAC TTA CAA AAC 1296
Ser Ile Glu Glu Val Asn Gly Lys Lys Leu Lys Glu Asn Leu Gln Asn
1295 1300 1305

TAT AAA GAG CAA GCA TTA ATG AGC AAA CAG CTT GCG ACA ATT CAT TGT 1344
Tyr Lys Glu Gln Ala Leu Met Ser Lys Gln Leu Ala Thr Ile His Cys
1310 1315 1320

GAA GCT CCT GTC GAA ATA AAA ATT CAA GAC CTT GAG TAT AAA GGC TAT 1392
Glu Ala Pro Val Glu Ile Lys Ile Gln Asp Leu Glu Tyr Lys Gly Tyr
1325 1330 1335 1340

GAC AAA GAA AAA GTA GTG AAA ATT TTT AAG GAA CTA GGC TTC CAA TCG 1440
Asp Lys Glu Lys Val Val Lys Ile Phe Lys Glu Leu Gly Phe Gln Ser
1345 1350 1355

CTC CTA GAC AAA ATG GGA GAG CAT GAG AAT GAA GAA GCG GAT GAA ATG 1488
Leu Leu Asp Lys Met Gly Glu His Glu Asn Glu Glu Ala Asp Glu Met
1360 1365 1370

CCG ACG ATT AAG TTC GAA AAA GTT GAA AAG CTG TCA GAC AAG GTT TTA 1536
Pro Thr Ile Lys Phe Glu Lys Val Glu Lys Leu Ser Asp Lys Val Leu
1375 1380 1385

TCA GAG AAG GCA GCT CTT TTA GTG GAA ATC ATT GAT GAA AAT TAT CAT 1584
Ser Glu Lys Ala Ala Leu Leu Val Glu Ile Ile Asp Glu Asn Tyr His
1390 1395 1400

ACT GGA GAA ATC ATC GGG TTT TCT ATC GCA AAC GAA AAT GGA TGT TTT 1632
Thr Gly Glu Ile Ile Gly Phe Ser Ile Ala Asn Glu Asn Gly Cys Phe
1405 1410 1415 1420

```
TAT ATT CCA GCC GAA ATT GCG CTA CAT TCA AAA GAG TTC ATA GAA TGG      1680
Tyr Ile Pro Ala Glu Ile Ala Leu His Ser Lys Glu Phe Ile Glu Trp
                1425                1430                1435

GTG AAG GAT GAA ACA AAG CGG AAA GTG GTG TAT GAT GCG AAA AAA TCA      1728
Val Lys Asp Glu Thr Lys Arg Lys Val Val Tyr Asp Ala Lys Lys Ser
            1440                1445                1450

ATT GTG GCG CTG CGC TGG CGA AAC ATT GAT TTA GCA GGT ATT GAG TTT      1776
Ile Val Ala Leu Arg Trp Arg Asn Ile Asp Leu Ala Gly Ile Glu Phe
        1455                1460                1465

GAT GTT CTC ATT GCC TCA TAC ATT TTA AAT CCG TCT GAA TCG ATT GAC      1824
Asp Val Leu Ile Ala Ser Tyr Ile Leu Asn Pro Ser Glu Ser Ile Asp
    1470                1475                1480

GAC ATA GCC GAG CTT GCC AAG ACA AAA AAT AAA CAT TTA GTT CAA AAG      1872
Asp Ile Ala Glu Leu Ala Lys Thr Lys Asn Lys His Leu Val Gln Lys
1485                1490                1495                1500

GAT GAA GTG ATT TAC GGA AAA GGC GCT AAA CGT CAT ATC CCT GAT GAA      1920
Asp Glu Val Ile Tyr Gly Lys Gly Ala Lys Arg His Ile Pro Asp Glu
                1505                1510                1515

GAC ATT TTA GGC GAA CAT CTT GCC AGA AAA GCG TTA GCC ATT TAT GAG      1968
Asp Ile Leu Gly Glu His Leu Ala Arg Lys Ala Leu Ala Ile Tyr Glu
            1520                1525                1530

CTG GAA GAA TTA TTA ATA CAA GAA TTA GAA GAA AAT GAA CAA TTT CAT      2016
Leu Glu Glu Leu Leu Ile Gln Glu Leu Glu Glu Asn Glu Gln Phe His
        1535                1540                1545

TTA TTC AGC GAA TTG GAG CTT CCG CTG TCA GCC ATT TTA TCT GAC ATG      2064
Leu Phe Ser Glu Leu Glu Leu Pro Leu Ser Ala Ile Leu Ser Asp Met
    1550                1555                1560

GAA ACA ACA GGA GTA AAG ATA GAC GTC AAC CGT CTG AAA GAA ATG GGA      2112
Glu Thr Thr Gly Val Lys Ile Asp Val Asn Arg Leu Lys Glu Met Gly
1565                1570                1575                1580

AAA GAG CTT GAT GAA CAG CTG AAG CAA TTA GAA AAG GAT ATT CAT CGT      2160
Lys Glu Leu Asp Glu Gln Leu Lys Gln Leu Glu Lys Asp Ile His Arg
                1585                1590                1595

CTA GCT GGA GTG TCA TTT AAC ATT AAT TCT CCG AAG CAG CTT GGG CCG      2208
Leu Ala Gly Val Ser Phe Asn Ile Asn Ser Pro Lys Gln Leu Gly Pro
            1600                1605                1610

ATT TTA TTT GAA AAG CTC AAT CTA CCG GTT TTG AAA AAG ACC AAA ACG      2256
Ile Leu Phe Glu Lys Leu Asn Leu Pro Val Leu Lys Lys Thr Lys Thr
        1615                1620                1625

GGG TAT TCG ACC TCT GCG GAC GTT TTA GAA AAA TTG AGA GGA CAG CAC      2304
Gly Tyr Ser Thr Ser Ala Asp Val Leu Glu Lys Leu Arg Gly Gln His
    1630                1635                1640

GAA ATT GTG GAG AAA ATT TTG CAT TAC CGG CAG CTC GGA AAG CTT CAA      2352
Glu Ile Val Glu Lys Ile Leu His Tyr Arg Gln Leu Gly Lys Leu Gln
1645                1650                1655                1660

TCG ACG TAT ATT GAA GGG CTG CTG AAG GTT GTC CAT CGT GAT ACG CAT      2400
Ser Thr Tyr Ile Glu Gly Leu Leu Lys Val Val His Arg Asp Thr His
```

1665 1670 1675

AAA ATC CAC ACC CGA TTT AAT CAA GCA TTA ACG CAA ACC GGA AGA TTA 2448
Lys Ile His Thr Arg Phe Asn Gln Ala Leu Thr Gln Thr Gly Arg Leu
1680 1685 1690

AGC TCC ACA GAC CCG AAT TTG CAA AAC ATT CCG ATT CGC CTT GAG GAA 2496
Ser Ser Thr Asp Pro Asn Leu Gln Asn Ile Pro Ile Arg Leu Glu Glu
1695 1700 1705

GGC CGC AAA ATT CGT CAA GCA TTT ATC CCT TCT GAA AAA GAT TGG GTC 2544
Gly Arg Lys Ile Arg Gln Ala Phe Ile Pro Ser Glu Lys Asp Trp Val
1710 1715 1720

ATT TTT GCA GCG GAC TAT TCC CAG ATT GAA CTG CGA GTG CTT GCG CAT 2592
Ile Phe Ala Ala Asp Tyr Ser Gln Ile Glu Leu Arg Val Leu Ala His
1725 1730 1735 1740

ATA TCT GGA GAT GAA AAA TTG ATT GAA GCG TTT AAA CAA GAT CTT GAT 2640
Ile Ser Gly Asp Glu Lys Leu Ile Glu Ala Phe Lys Gln Asp Leu Asp
1745 1750 1755

ATT CAT ACA AAA ACG GCG ATC GAT GTG TTC CAT GTC GAA GAA GAT AAA 2688
Ile His Thr Lys Thr Ala Ile Asp Val Phe His Val Glu Glu Asp Lys
1760 1765 1770

GTG ACC TCC AAC ATG AGA AGA CAG GCA AAA GCA GTT AAT TTC GGG ATT 2736
Val Thr Ser Asn Met Arg Arg Gln Ala Lys Ala Val Asn Phe Gly Ile
1775 1780 1785

GTT TAC GGA ATC AGC GAT TAC GGA TTG TCG CAA AAC TTA GGA ATT ACC 2784
Val Tyr Gly Ile Ser Asp Tyr Gly Leu Ser Gln Asn Leu Gly Ile Thr
1790 1795 1800

CGA AAA GAA GCT GGT GAA TTT ATT AAA AAA TAT TTT GAA ATT TAT AAA 2832
Arg Lys Glu Ala Gly Glu Phe Ile Lys Lys Tyr Phe Glu Ile Tyr Lys
1805 1810 1815 1820

GGC GTT AAA GAA TAT ATG GAT GGC ATA ATC CAA GAG GCG AAG CAA AAA 2880
Gly Val Lys Glu Tyr Met Asp Gly Ile Ile Gln Glu Ala Lys Gln Lys
1825 1830 1835

GGC TAT GTA ACG ACA CTA ATG CAG CGT CGG AGA TAT ATT CCG GAA ATT 2928
Gly Tyr Val Thr Thr Leu Met Gln Arg Arg Arg Tyr Ile Pro Glu Ile
1840 1845 1850

ACG AGC AGA AAT TTC AAT ATC AGA AGC TTC GCT GAG CGA ACA GCC ATG 2976
Thr Ser Arg Asn Phe Asn Ile Arg Ser Phe Ala Glu Arg Thr Ala Met
1855 1860 1865

AAT ACT CCG ATT CAA GGA AGT GCA GCG GAT ATT ATC AAA AAA GCG ATG 3024
Asn Thr Pro Ile Gln Gly Ser Ala Ala Asp Ile Ile Lys Lys Ala Met
1870 1875 1880

ATC GAT ATG GCG CAA GAA ATT GAA AAA CGA AAT TTG CAA ACG AGG CTG 3072
Ile Asp Met Ala Gln Glu Ile Glu Lys Arg Asn Leu Gln Thr Arg Leu
1885 1890 1895 1900

CTG CTT CAA GTT CAT GAC GAA TTG GTG TTT GAA GCG CCA AAG GAT GAA 3120
Leu Leu Gln Val His Asp Glu Leu Val Phe Glu Ala Pro Lys Asp Glu
1905 1910 1915

ATT GAA ATT TTA GAA AAG CTT GTT CCG GAA GTA ATG GAA AAT GCC ATT 3168

```
Ile Glu Ile Leu Glu Lys Leu Val Pro Glu Val Met Glu Asn Ala Ile
            1920                1925                1930

CAG CTA AAA GTA CCG TTA AAG GTT GAT TAT TCT TAC GGT TCT ACG TGG      3216
Gln Leu Lys Val Pro Leu Lys Val Asp Tyr Ser Tyr Gly Ser Thr Trp
        1935                1940                1945

TAT GAC GCC AAA TCA TCT CAT CAT CAT CAT CAT CAT TAA                  3255
Tyr Asp Ala Lys Ser Ser His His His His His His
    1950                1955                1960
```

(2) INFORMATION FOR SEQ ID NO:6:

(i) SEQUENCE CHARACTERISTICS:

(A) LENGTH: 1084 amino acids
(B) TYPE: amino acid
(D) TOPOLOGY: linear

(ii) MOLECULE TYPE: protein

(xi) SEQUENCE DESCRIPTION: SEQ ID NO:6:

Met Lys Ile Glu Glu Gly Lys Leu Val Ile Trp Ile Asn Gly Asp Lys
1 5 10 15

Gly Tyr Asn Gly Leu Ala Glu Val Gly Lys Lys Phe Glu Lys Asp Thr
20 25 30

Gly Ile Lys Val Thr Val Glu His Pro Asp Lys Leu Glu Glu Lys Phe
35 40 45

Pro Gln Val Ala Ala Thr Gly Asp Gly Pro Asp Ile Ile Phe Trp Ala
50 55 60

His Asp Arg Phe Gly Gly Tyr Ala Gln Ser Gly Leu Leu Ala Glu Ile
65 70 75 80

Thr Pro Asp Lys Ala Phe Gln Asp Lys Leu Tyr Pro Phe Thr Trp Asp
85 90 95

Ala Val Arg Tyr Asn Gly Lys Leu Ile Ala Tyr Pro Ile Ala Val Glu
100 105 110

Ala Leu Ser Leu Ile Tyr Asn Lys Asp Leu Leu Pro Asn Pro Pro Lys
115 120 125

Thr Trp Glu Glu Ile Pro Ala Leu Asp Lys Glu Leu Lys Ala Lys Gly
130 135 140

Lys Ser Ala Leu Met Phe Asn Leu Gln Glu Pro Tyr Phe Thr Trp Pro
145 150 155 160

Leu Ile Ala Ala Asp Gly Gly Tyr Ala Phe Lys Tyr Glu Asn Gly Lys
165 170 175

Tyr Asp Ile Lys Asp Val Gly Val Asp Asn Ala Gly Ala Lys Ala Gly
180 185 190

Leu Thr Phe Leu Val Asp Leu Ile Lys Asn Lys His Met Asn Ala Asp
195 200 205

Thr Asp Tyr Ser Ile Ala Glu Ala Ala Phe Asn Lys Gly Glu Thr Ala

210 215 220

Met Thr Ile Asn Gly Pro Trp Ala Trp Ser Asn Ile Asp Thr Ser Lys
225 230 235 240

Val Asn Tyr Gly Val Thr Val Leu Pro Thr Phe Lys Gly Gln Pro Ser
245 250 255

Lys Pro Phe Val Gly Val Leu Ser Ala Gly Ile Asn Ala Ala Ser Pro
260 265 270

Asn Lys Glu Leu Ala Lys Glu Phe Leu Glu Asn Tyr Leu Leu Thr Asp
275 280 285

Glu Gly Leu Glu Ala Val Asn Lys Asp Lys Pro Leu Gly Ala Val Ala
290 295 300

Leu Lys Ser Tyr Glu Glu Glu Leu Ala Lys Asp Pro Arg Ile Ala Ala
305 310 315 320

Thr Met Glu Asn Ala Gln Lys Gly Glu Ile Met Pro Asn Ile Pro Gln
325 330 335

Met Ser Ala Phe Trp Tyr Ala Val Arg Thr Ala Val Ile Asn Ala Ala
340 345 350

Ser Gly Arg Gln Thr Val Asp Glu Ala Leu Lys Asp Ala Gln Thr Asn
355 360 365

Ser Ser Ser Asn Asn Asn Asn Asn Asn Asn Asn Asn Asn Leu Gly Ile
370 375 380

Glu Gly Arg Ile Ser Glu Phe Gly Val Pro Gly Val Gly Glu Lys Thr
385 390 395 400

Ala Ile Lys Leu Leu Lys Gln Phe Glu Thr Val Glu Asn Ile Leu Asn
405 410 415

Ser Ile Glu Glu Val Asn Gly Lys Lys Leu Lys Glu Asn Leu Gln Asn
420 425 430

Tyr Lys Glu Gln Ala Leu Met Ser Lys Gln Leu Ala Thr Ile His Cys
435 440 445

Glu Ala Pro Val Glu Ile Lys Ile Gln Asp Leu Glu Tyr Lys Gly Tyr
450 455 460

Asp Lys Glu Lys Val Val Lys Ile Phe Lys Glu Leu Gly Phe Gln Ser
465 470 475 480

Leu Leu Asp Lys Met Gly Glu His Glu Asn Glu Glu Ala Asp Glu Met
485 490 495

Pro Thr Ile Lys Phe Glu Lys Val Glu Lys Leu Ser Asp Lys Val Leu
500 505 510

Ser Glu Lys Ala Ala Leu Leu Val Glu Ile Ile Asp Glu Asn Tyr His
515 520 525

Thr Gly Glu Ile Ile Gly Phe Ser Ile Ala Asn Glu Asn Gly Cys Phe
530 535 540

Tyr Ile Pro Ala Glu Ile Ala Leu His Ser Lys Glu Phe Ile Glu Trp

545 550 555 560

Val Lys Asp Glu Thr Lys Arg Lys Val Val Tyr Asp Ala Lys Lys Ser
565 570 575

Ile Val Ala Leu Arg Trp Arg Asn Ile Asp Leu Ala Gly Ile Glu Phe
580 585 590

Asp Val Leu Ile Ala Ser Tyr Ile Leu Asn Pro Ser Glu Ser Ile Asp
595 600 605

Asp Ile Ala Glu Leu Ala Lys Thr Lys Asn Lys His Leu Val Gln Lys
610 615 620

Asp Glu Val Ile Tyr Gly Lys Gly Ala Lys Arg His Ile Pro Asp Glu
625 630 635 640

Asp Ile Leu Gly Glu His Leu Ala Arg Lys Ala Leu Ala Ile Tyr Glu
645 650 655

Leu Glu Glu Leu Leu Ile Gln Glu Leu Glu Glu Asn Glu Gln Phe His
660 665 670

Leu Phe Ser Glu Leu Glu Leu Pro Leu Ser Ala Ile Leu Ser Asp Met
675 680 685

Glu Thr Thr Gly Val Lys Ile Asp Val Asn Arg Leu Lys Glu Met Gly
690 695 700

Lys Glu Leu Asp Glu Gln Leu Lys Gln Leu Glu Lys Asp Ile His Arg
705 710 715 720

Leu Ala Gly Val Ser Phe Asn Ile Asn Ser Pro Lys Gln Leu Gly Pro
725 730 735

Ile Leu Phe Glu Lys Leu Asn Leu Pro Val Leu Lys Lys Thr Lys Thr
740 745 750

Gly Tyr Ser Thr Ser Ala Asp Val Leu Glu Lys Leu Arg Gly Gln His
755 760 765

Glu Ile Val Glu Lys Ile Leu His Tyr Arg Gln Leu Gly Lys Leu Gln
770 775 780

Ser Thr Tyr Ile Glu Gly Leu Leu Lys Val Val His Arg Asp Thr His
785 790 795 800

Lys Ile His Thr Arg Phe Asn Gln Ala Leu Thr Gln Thr Gly Arg Leu
805 810 815

Ser Ser Thr Asp Pro Asn Leu Gln Asn Ile Pro Ile Arg Leu Glu Glu
820 825 830

Gly Arg Lys Ile Arg Gln Ala Phe Ile Pro Ser Glu Lys Asp Trp Val
835 840 845

Ile Phe Ala Ala Asp Tyr Ser Gln Ile Glu Leu Arg Val Leu Ala His
850 855 860

Ile Ser Gly Asp Glu Lys Leu Ile Glu Ala Phe Lys Gln Asp Leu Asp
865 870 875 880

Ile His Thr Lys Thr Ala Ile Asp Val Phe His Val Glu Glu Asp Lys

885 890 895

Val Thr Ser Asn Met Arg Arg Gln Ala Lys Ala Val Asn Phe Gly Ile
900 905 910

Val Tyr Gly Ile Ser Asp Tyr Gly Leu Ser Gln Asn Leu Gly Ile Thr
915 920 925

Arg Lys Glu Ala Gly Glu Phe Ile Lys Lys Tyr Phe Glu Ile Tyr Lys
930 935 940

Gly Val Lys Glu Tyr Met Asp Gly Ile Ile Gln Glu Ala Lys Gln Lys
945 950 955 960

Gly Tyr Val Thr Thr Leu Met Gln Arg Arg Arg Tyr Ile Pro Glu Ile
965 970 975

Thr Ser Arg Asn Phe Asn Ile Arg Ser Phe Ala Glu Arg Thr Ala Met
980 985 990

Asn Thr Pro Ile Gln Gly Ser Ala Ala Asp Ile Ile Lys Lys Ala Met
995 1000 1005

Ile Asp Met Ala Gln Glu Ile Glu Lys Arg Asn Leu Gln Thr Arg Leu
1010 1015 1020

Leu Leu Gln Val His Asp Glu Leu Val Phe Glu Ala Pro Lys Asp Glu
1025 1030 1035 1040

Ile Glu Ile Leu Glu Lys Leu Val Pro Glu Val Met Glu Asn Ala Ile
1045 1050 1055

Gln Leu Lys Val Pro Leu Lys Val Asp Tyr Ser Tyr Gly Ser Thr Trp
1060 1065 1070

Tyr Asp Ala Lys Ser Ser His His His His His His
1075 1080

(2) INFORMATION FOR SEQ ID NO:7:

(i) SEQUENCE CHARACTERISTICS:

(A) LENGTH: 3831 base pairs
(B) TYPE: nucleic acid
(C) STRANDEDNESS: double
(D) TOPOLOGY: linear

(ix) FEATURE:

(A) NAME/KEY: CDS
(B) LOCATION: 1..3828

(xi) SEQUENCE DESCRIPTION: SEQ ID NO:7:

```
ATG AAA ATC GAA GAA GGT AAA CTG GTA ATC TGG ATT AAC GGC GAT AAA          48
Met Lys Ile Glu Glu Gly Lys Leu Val Ile Trp Ile Asn Gly Asp Lys
1085                1090                1095                1100

GGC TAT AAC GGT CTC GCT GAA GTC GGT AAG AAA TTC GAG AAA GAT ACC          96
Gly Tyr Asn Gly Leu Ala Glu Val Gly Lys Lys Phe Glu Lys Asp Thr
                1105                1110                1115
```

GGA ATT AAA GTC ACC GTT GAG CAT CCG GAT AAA CTG GAA GAG AAA TTC 144
Gly Ile Lys Val Thr Val Glu His Pro Asp Lys Leu Glu Glu Lys Phe
1120 1125 1130

CCA CAG GTT GCG GCA ACT GGC GAT GGC CCT GAC ATT ATC TTC TGG GCA 192
Pro Gln Val Ala Ala Thr Gly Asp Gly Pro Asp Ile Ile Phe Trp Ala
1135 1140 1145

CAC GAC CGC TTT GGT GGC TAC GCT CAA TCT GGC CTG TTG GCT GAA ATC 240
His Asp Arg Phe Gly Gly Tyr Ala Gln Ser Gly Leu Leu Ala Glu Ile
1150 1155 1160

ACC CCG GAC AAA GCG TTC CAG GAC AAG CTG TAT CCG TTT ACC TGG GAT 288
Thr Pro Asp Lys Ala Phe Gln Asp Lys Leu Tyr Pro Phe Thr Trp Asp
1165 1170 1175 1180

GCC GTA CGT TAC AAC GGC AAG CTG ATT GCT TAC CCG ATC GCT GTT GAA 336
Ala Val Arg Tyr Asn Gly Lys Leu Ile Ala Tyr Pro Ile Ala Val Glu
1185 1190 1195

GCG TTA TCG CTG ATT TAT AAC AAA GAT CTG CTG CCG AAC CCG CCA AAA 384
Ala Leu Ser Leu Ile Tyr Asn Lys Asp Leu Leu Pro Asn Pro Pro Lys
1200 1205 1210

ACC TGG GAA GAG ATC CCG GCG CTG GAT AAA GAA CTG AAA GCG AAA GGT 432
Thr Trp Glu Glu Ile Pro Ala Leu Asp Lys Glu Leu Lys Ala Lys Gly
1215 1220 1225

AAG AGC GCG CTG ATG TTC AAC CTG CAA GAA CCG TAC TTC ACC TGG CCG 480
Lys Ser Ala Leu Met Phe Asn Leu Gln Glu Pro Tyr Phe Thr Trp Pro
1230 1235 1240

CTG ATT GCT GCT GAC GGG GGT TAT GCG TTC AAG TAT GAA AAC GGC AAG 528
Leu Ile Ala Ala Asp Gly Gly Tyr Ala Phe Lys Tyr Glu Asn Gly Lys
1245 1250 1255 1260

TAC GAC ATT AAA GAC GTG GGC GTG GAT AAC GCT GGC GCG AAA GCG GGT 576
Tyr Asp Ile Lys Asp Val Gly Val Asp Asn Ala Gly Ala Lys Ala Gly
1265 1270 1275

CTG ACC TTC CTG GTT GAC CTG ATT AAA AAC AAA CAC ATG AAT GCA GAC 624
Leu Thr Phe Leu Val Asp Leu Ile Lys Asn Lys His Met Asn Ala Asp
1280 1285 1290

ACC GAT TAC TCC ATC GCA GAA GCT GCC TTT AAT AAA GGC GAA ACA GCG 672
Thr Asp Tyr Ser Ile Ala Glu Ala Ala Phe Asn Lys Gly Glu Thr Ala
1295 1300 1305

ATG ACC ATC AAC GGC CCG TGG GCA TGG TCC AAC ATC GAC ACC AGC AAA 720
Met Thr Ile Asn Gly Pro Trp Ala Trp Ser Asn Ile Asp Thr Ser Lys
1310 1315 1320

GTG AAT TAT GGT GTA ACG GTA CTG CCG ACC TTC AAG GGT CAA CCA TCC 768
Val Asn Tyr Gly Val Thr Val Leu Pro Thr Phe Lys Gly Gln Pro Ser
1325 1330 1335 1340

AAA CCG TTC GTT GGC GTG CTG AGC GCA GGT ATT AAC GCC GCC AGT CCG 816
Lys Pro Phe Val Gly Val Leu Ser Ala Gly Ile Asn Ala Ala Ser Pro
1345 1350 1355

AAC AAA GAG CTG GCA AAA GAG TTC CTC GAA AAC TAT CTG CTG ACT GAT 864
Asn Lys Glu Leu Ala Lys Glu Phe Leu Glu Asn Tyr Leu Leu Thr Asp
1360 1365 1370

```
GAA GGT CTG GAA GCG GTT AAT AAA GAC AAA CCG CTG GGT GCC GTA GCG      912
Glu Gly Leu Glu Ala Val Asn Lys Asp Lys Pro Leu Gly Ala Val Ala
        1375                1380                1385

CTG AAG TCT TAC GAG GAA GAG TTG GCG AAA GAT CCA CGT ATT GCC GCC      960
Leu Lys Ser Tyr Glu Glu Glu Leu Ala Lys Asp Pro Arg Ile Ala Ala
    1390                1395                1400

ACC ATG GAA AAC GCC CAG AAA GGT GAA ATC ATG CCG AAC ATC CCG CAG     1008
Thr Met Glu Asn Ala Gln Lys Gly Glu Ile Met Pro Asn Ile Pro Gln
1405                1410                1415                1420

ATG TCC GCT TTC TGG TAT GCC GTG CGT ACT GCG GTG ATC AAC GCC GCC     1056
Met Ser Ala Phe Trp Tyr Ala Val Arg Thr Ala Val Ile Asn Ala Ala
                1425                1430                1435

AGC GGT CGT CAG ACT GTC GAT GAA GCC CTG AAA GAC GCG CAG ACT AAT     1104
Ser Gly Arg Gln Thr Val Asp Glu Ala Leu Lys Asp Ala Gln Thr Asn
            1440                1445                1450

TCG AGC TCG AAC AAC AAC AAC AAT AAC AAT AAC AAC AAC CTC GGG ATC     1152
Ser Ser Ser Asn Asn Asn Asn Asn Asn Asn Asn Asn Asn Leu Gly Ile
        1455                1460                1465

GAG GGA AGG ATT TCA GAA TTC GGC GTG ACA AAG AAG CTA GTT TTA ATT     1200
Glu Gly Arg Ile Ser Glu Phe Gly Val Thr Lys Lys Leu Val Leu Ile
    1470                1475                1480

GAT GGA AAC AGT ATT GCT TAC AGA GCG TTT TTC GCT TTG CCG CTT TTA     1248
Asp Gly Asn Ser Ile Ala Tyr Arg Ala Phe Phe Ala Leu Pro Leu Leu
1485                1490                1495                1500

AAT AAT GAT AAG GGG ATT TAT ACG AAT GCA ATT TAC GGC TTT ACA AAT     1296
Asn Asn Asp Lys Gly Ile Tyr Thr Asn Ala Ile Tyr Gly Phe Thr Asn
                1505                1510                1515

ATG CTG TTA AAA GTA CTG GAG GAA GAA AAA CCG ACA CAT ATT CTT GTT     1344
Met Leu Leu Lys Val Leu Glu Glu Glu Lys Pro Thr His Ile Leu Val
            1520                1525                1530

GCA TTT GAT GCA GGG AAA ACA ACG TTC CGG CAT AAA ACT TTT AAA GAA     1392
Ala Phe Asp Ala Gly Lys Thr Thr Phe Arg His Lys Thr Phe Lys Glu
        1535                1540                1545

TAT AAA GGA ACT CGG CAA AAA ACC CCG CCT GAA TTG TCG GAG CAG CTA     1440
Tyr Lys Gly Thr Arg Gln Lys Thr Pro Pro Glu Leu Ser Glu Gln Leu
    1550                1555                1560

CCA TTT ATA CGG GAT TTG CTT GAT GCC TAC CAA ATT ACA ACA TAT GAA     1488
Pro Phe Ile Arg Asp Leu Leu Asp Ala Tyr Gln Ile Thr Thr Tyr Glu
1565                1570                1575                1580

CTC GAA AAT TAT GAG GCT GAT GAT ATT ATT GGA ACA GTT GCG AGA CAA     1536
Leu Glu Asn Tyr Glu Ala Asp Asp Ile Ile Gly Thr Val Ala Arg Gln
                1585                1590                1595

GCT GAG AAG CAA GAT TTT GAA GTG AAA ATT ATT TCC GGA GAT AAG GAT     1584
Ala Glu Lys Gln Asp Phe Glu Val Lys Ile Ile Ser Gly Asp Lys Asp
            1600                1605                1610

TTA ACA CAG CTG GCA ACT GAA AAA ACG ACC GTT TCC ATC ACG AAA AAA     1632
Leu Thr Gln Leu Ala Thr Glu Lys Thr Thr Val Ser Ile Thr Lys Lys
```

```
            1615                1620                1625

GGA ATT ACA GAT GTT GAA CCG CAC ACG CCT GAA TCG ATT CAA GAG AAG      1680
Gly Ile Thr Asp Val Glu Pro His Thr Pro Glu Ser Ile Gln Glu Lys
        1630                1635                1640

TAT GGG CTA AGC CCG GCA CAA ATT ATT GAT TTG AAA GGA TTG ATG GGC      1728
Tyr Gly Leu Ser Pro Ala Gln Ile Ile Asp Leu Lys Gly Leu Met Gly
1645                1650                1655                1660

GAT CAA TCA GAT AAT ATC CCA GGT GTG CCC GGC GTT GGA GAG AAA ACC      1776
Asp Gln Ser Asp Asn Ile Pro Gly Val Pro Gly Val Gly Glu Lys Thr
                1665                1670                1675

GCG ATT AAA TTG CTG AAA CAG TTT GAG ACA GTC GAA AAT ATT TTA AAT      1824
Ala Ile Lys Leu Leu Lys Gln Phe Glu Thr Val Glu Asn Ile Leu Asn
            1680                1685                1690

TCG ATT GAA GAA GTA AAT GGA AAA AAA TTG AAG GAA AAC TTA CAA AAC      1872
Ser Ile Glu Glu Val Asn Gly Lys Lys Leu Lys Glu Asn Leu Gln Asn
        1695                1700                1705

TAT AAA GAG CAA GCA TTA ATG AGC AAA CAG CTT GCG ACA ATT CAT TGT      1920
Tyr Lys Glu Gln Ala Leu Met Ser Lys Gln Leu Ala Thr Ile His Cys
    1710                1715                1720

GAA GCT CCT GTC GAA ATA AAA ATT CAA GAC CTT GAG TAT AAA GGC TAT      1968
Glu Ala Pro Val Glu Ile Lys Ile Gln Asp Leu Glu Tyr Lys Gly Tyr
1725                1730                1735                1740

GAC AAA GAA AAA GTA GTG AAA ATT TTT AAG GAA CTA GGC TTC CAA TCG      2016
Asp Lys Glu Lys Val Val Lys Ile Phe Lys Glu Leu Gly Phe Gln Ser
                1745                1750                1755

CTC CTA GAC AAA ATG GGA GAG CAT GAG AAT GAA GAA GCG GAT GAA ATG      2064
Leu Leu Asp Lys Met Gly Glu His Glu Asn Glu Glu Ala Asp Glu Met
            1760                1765                1770

CCG ACG ATT AAG TTC GAA AAA GTT GAA AAG CTG TCA GAC AAG GTT TTA      2112
Pro Thr Ile Lys Phe Glu Lys Val Glu Lys Leu Ser Asp Lys Val Leu
        1775                1780                1785

TCA GAG AAG GCA GCT CTT TTA GTG GAA ATC ATT GAT GAA AAT TAT CAT      2160
Ser Glu Lys Ala Ala Leu Leu Val Glu Ile Ile Asp Glu Asn Tyr His
    1790                1795                1800

ACT GGA GAA ATC ATC GGG TTT TCT ATC GCA AAC GAA AAT GGA TGT TTT      2208
Thr Gly Glu Ile Ile Gly Phe Ser Ile Ala Asn Glu Asn Gly Cys Phe
1805                1810                1815                1820

TAT ATT CCA GCC GAA ATT GCG CTA CAT TCA AAA GAG TTC ATA GAA TGG      2256
Tyr Ile Pro Ala Glu Ile Ala Leu His Ser Lys Glu Phe Ile Glu Trp
                1825                1830                1835

GTG AAG GAT GAA ACA AAG CGG AAA GTG GTG TAT GAT GCG AAA AAA TCA      2304
Val Lys Asp Glu Thr Lys Arg Lys Val Val Tyr Asp Ala Lys Lys Ser
            1840                1845                1850

ATT GTG GCG CTG CGC TGG CGA AAC ATT GAT TTA GCA GGT ATT GAG TTT      2352
Ile Val Ala Leu Arg Trp Arg Asn Ile Asp Leu Ala Gly Ile Glu Phe
        1855                1860                1865

GAT GTT CTC ATT GCC TCA TAC ATT TTA AAT CCG TCT GAA TCG ATT GAC      2400
```

```
Asp Val Leu Ile Ala Ser Tyr Ile Leu Asn Pro Ser Glu Ser Ile Asp
    1870                1875                1880

GAC ATA GCC GAG CTT GCC AAG ACA AAA AAT AAA CAT TTA GTT CAA AAG      2448
Asp Ile Ala Glu Leu Ala Lys Thr Lys Asn Lys His Leu Val Gln Lys
1885                1890                1895                1900

GAT GAA GTG ATT TAC GGA AAA GGC GCT AAA CGT CAT ATC CCT GAT GAA      2496
Asp Glu Val Ile Tyr Gly Lys Gly Ala Lys Arg His Ile Pro Asp Glu
                1905                1910                1915

GAC ATT TTA GGC GAA CAT CTT GCC AGA AAA GCG TTA GCC ATT TAT GAG      2544
Asp Ile Leu Gly Glu His Leu Ala Arg Lys Ala Leu Ala Ile Tyr Glu
            1920                1925                1930

CTG GAA GAA TTA TTA ATA CAA GAA TTA GAA GAA AAT GAA CAA TTT CAT      2592
Leu Glu Glu Leu Leu Ile Gln Glu Leu Glu Glu Asn Glu Gln Phe His
        1935                1940                1945

TTA TTC AGC GAA TTG GAG CTT CCG CTG TCA GCC ATT TTA TCT GAC ATG      2640
Leu Phe Ser Glu Leu Glu Leu Pro Leu Ser Ala Ile Leu Ser Asp Met
    1950                1955                1960

GAA ACA ACA GGA GTA AAG ATA GAC GTC AAC CGT CTG AAA GAA ATG GGA      2688
Glu Thr Thr Gly Val Lys Ile Asp Val Asn Arg Leu Lys Glu Met Gly
1965                1970                1975                1980

AAA GAG CTT GAT GAA CAG CTG AAG CAA TTA GAA AAG GAT ATT CAT CGT      2736
Lys Glu Leu Asp Glu Gln Leu Lys Gln Leu Glu Lys Asp Ile His Arg
                1985                1990                1995

CTA GCT GGA GTG TCA TTT AAC ATT AAT TCT CCG AAG CAG CTT GGG CCG      2784
Leu Ala Gly Val Ser Phe Asn Ile Asn Ser Pro Lys Gln Leu Gly Pro
            2000                2005                2010

ATT TTA TTT GAA AAG CTC AAT CTA CCG GTT TTG AAA AAG ACC AAA ACG      2832
Ile Leu Phe Glu Lys Leu Asn Leu Pro Val Leu Lys Lys Thr Lys Thr
        2015                2020                2025

GGG TAT TCG ACC TCT GCG GAC GTT TTA GAA AAA TTG AGA GGA CAG CAC      2880
Gly Tyr Ser Thr Ser Ala Asp Val Leu Glu Lys Leu Arg Gly Gln His
    2030                2035                2040

GAA ATT GTG GAG AAA ATT TTG CAT TAC CGG CAG CTC GGA AAG CTT CAA      2928
Glu Ile Val Glu Lys Ile Leu His Tyr Arg Gln Leu Gly Lys Leu Gln
2045                2050                2055                2060

TCG ACG TAT ATT GAA GGG CTG CTG AAG GTT GTC CAT CGT GAT ACG CAT      2976
Ser Thr Tyr Ile Glu Gly Leu Leu Lys Val Val His Arg Asp Thr His
                2065                2070                2075

AAA ATC CAC ACC CGA TTT AAT CAA GCA TTA ACG CAA ACC GGA AGA TTA      3024
Lys Ile His Thr Arg Phe Asn Gln Ala Leu Thr Gln Thr Gly Arg Leu
            2080                2085                2090

AGC TCC ACA GAC CCG AAT TTG CAA AAC ATT CCG ATT CGC CTT GAG GAA      3072
Ser Ser Thr Asp Pro Asn Leu Gln Asn Ile Pro Ile Arg Leu Glu Glu
        2095                2100                2105

GGC CGC AAA ATT CGT CAA GCA TTT ATC CCT TCT GAA AAA GAT TGG GTC      3120
Gly Arg Lys Ile Arg Gln Ala Phe Ile Pro Ser Glu Lys Asp Trp Val
    2110                2115                2120
```

```
ATT TTT GCA GCG GAC TAT TCC CAG ATT GAA CTG CGA GTG CTT GGG CAT      3168
Ile Phe Ala Ala Asp Tyr Ser Gln Ile Glu Leu Arg Val Leu Ala His
2125                2130                2135                2140

ATA TCT GGA GAT GAA AAA TTG ATT GAA GCG TTT AAA CAA GAT CTT GAT      3216
Ile Ser Gly Asp Glu Lys Leu Ile Glu Ala Phe Lys Gln Asp Leu Asp
                2145                2150                2155

ATT CAT ACA AAA ACG GCG ATC GAT GTG TTC CAT GTC GAA GAA GAT AAA      3264
Ile His Thr Lys Thr Ala Ile Asp Val Phe His Val Glu Glu Asp Lys
            2160                2165                2170

GTG ACC TCC AAC ATG AGA AGA CAG GCA AAA GCA GTT AAT TTC GGG ATT      3312
Val Thr Ser Asn Met Arg Arg Gln Ala Lys Ala Val Asn Phe Gly Ile
        2175                2180                2185

GTT TAC GGA ATC AGC GAT TAC GGA TTG TCG CAA AAC TTA GGA ATT ACC      3360
Val Tyr Gly Ile Ser Asp Tyr Gly Leu Ser Gln Asn Leu Gly Ile Thr
    2190                2195                2200

CGA AAA GAA GCT GGT GAA TTT ATT AAA AAA TAT TTT GAA ATT TAT AAA      3408
Arg Lys Glu Ala Gly Glu Phe Ile Lys Lys Tyr Phe Glu Ile Tyr Lys
2205                2210                2215                2220

GGC GTT AAA GAA TAT ATG GAT GGC ATA ATC CAA GAG GCG AAG CAA AAA      3456
Gly Val Lys Glu Tyr Met Asp Gly Ile Ile Gln Glu Ala Lys Gln Lys
                2225                2230                2235

GGC TAT GTA ACG ACA CTA ATG CAG CGT CGG AGA TAT ATT CCG GAA ATT      3504
Gly Tyr Val Thr Thr Leu Met Gln Arg Arg Arg Tyr Ile Pro Glu Ile
            2240                2245                2250

ACG AGC AGA AAT TTC AAT ATC AGA AGC TTC GCT GAG CGA ACA GCC ATG      3552
Thr Ser Arg Asn Phe Asn Ile Arg Ser Phe Ala Glu Arg Thr Ala Met
        2255                2260                2265

AAT ACT CCG ATT CAA GGA AGT GCA GCG GAT ATT ATC AAA AAA GCG ATG      3600
Asn Thr Pro Ile Gln Gly Ser Ala Ala Asp Ile Ile Lys Lys Ala Met
    2270                2275                2280

ATC GAT ATG GCG CAA GAA ATT GAA AAA CGA AAT TTG CAA ACG AGG CTG      3648
Ile Asp Met Ala Gln Glu Ile Glu Lys Arg Asn Leu Gln Thr Arg Leu
2285                2290                2295                2300

CTG CTT CAA GTT CAT GAC GAA TTG GTG TTT GAA GCG CCA AAG GAT GAA      3696
Leu Leu Gln Val His Asp Glu Leu Val Phe Glu Ala Pro Lys Asp Glu
                2305                2310                2315

ATT GAA ATT TTA GAA AAG CTT GTT CCG GAA GTA ATG GAA AAT GCC ATT      3744
Ile Glu Ile Leu Glu Lys Leu Val Pro Glu Val Met Glu Asn Ala Ile
            2320                2325                2330

CAG CTA AAA GTA CCG TTA AAG GTT GAT TAT TCT TAC GGT TCT ACG TGG      3792
Gln Leu Lys Val Pro Leu Lys Val Asp Tyr Ser Tyr Gly Ser Thr Trp
        2335                2340                2345

TAT GAC GCC AAA TCA TCT CAT CAT CAT CAT CAT CAT TAA                  3831
Tyr Asp Ala Lys Ser Ser His His His His His His
    2350                2355                2360
```

(2) INFORMATION FOR SEQ ID NO:8:

(i) SEQUENCE CHARACTERISTICS:

(A) LENGTH: 1276 amino acids
(B) TYPE: amino acid
(D) TOPOLOGY: linear

(ii) MOLECULE TYPE: protein

(xi) SEQUENCE DESCRIPTION: SEQ ID NO:8:

```
Met Lys Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285
```

Glu Gly Leu Glu Ala Val Asn Lys Asp Lys Pro Leu Gly Ala Val Ala
290 295 300

Leu Lys Ser Tyr Glu Glu Glu Leu Ala Lys Asp Pro Arg Ile Ala Ala
305 310 315 320

Thr Met Glu Asn Ala Gln Lys Gly Glu Ile Met Pro Asn Ile Pro Gln
325 330 335

Met Ser Ala Phe Trp Tyr Ala Val Arg Thr Ala Val Ile Asn Ala Ala
340 345 350

Ser Gly Arg Gln Thr Val Asp Glu Ala Leu Lys Asp Ala Gln Thr Asn
355 360 365

Ser Ser Ser Asn Asn Asn Asn Asn Asn Asn Asn Asn Asn Leu Gly Ile
370 375 380

Glu Gly Arg Ile Ser Glu Phe Gly Val Thr Lys Lys Leu Val Leu Ile
385 390 395 400

Asp Gly Asn Ser Ile Ala Tyr Arg Ala Phe Phe Ala Leu Pro Leu Leu
405 410 415

Asn Asn Asp Lys Gly Ile Tyr Thr Asn Ala Ile Tyr Gly Phe Thr Asn
420 425 430

Met Leu Leu Lys Val Leu Glu Glu Glu Lys Pro Thr His Ile Leu Val
435 440 445

Ala Phe Asp Ala Gly Lys Thr Thr Phe Arg His Lys Thr Phe Lys Glu
450 455 460

Tyr Lys Gly Thr Arg Gln Lys Thr Pro Pro Glu Leu Ser Glu Gln Leu
465 470 475 480

Pro Phe Ile Arg Asp Leu Leu Asp Ala Tyr Gln Ile Thr Thr Tyr Glu
485 490 495

Leu Glu Asn Tyr Glu Ala Asp Asp Ile Ile Gly Thr Val Ala Arg Gln
500 505 510

Ala Glu Lys Gln Asp Phe Glu Val Lys Ile Ile Ser Gly Asp Lys Asp
515 520 525

Leu Thr Gln Leu Ala Thr Glu Lys Thr Thr Val Ser Ile Thr Lys Lys
530 535 540

Gly Ile Thr Asp Val Glu Pro His Thr Pro Glu Ser Ile Gln Glu Lys
545 550 555 560

Tyr Gly Leu Ser Pro Ala Gln Ile Ile Asp Leu Lys Gly Leu Met Gly
565 570 575

Asp Gln Ser Asp Asn Ile Pro Gly Val Pro Gly Val Gly Glu Lys Thr
580 585 590

Ala Ile Lys Leu Leu Lys Gln Phe Glu Thr Val Glu Asn Ile Leu Asn
595 600 605

Ser Ile Glu Glu Val Asn Gly Lys Lys Leu Lys Glu Asn Leu Gln Asn
610 615 620

Tyr Lys Glu Gln Ala Leu Met Ser Lys Gln Leu Ala Thr Ile His Cys
625 630 635 640

Glu Ala Pro Val Glu Ile Lys Ile Gln Asp Leu Glu Tyr Lys Gly Tyr
645 650 655

Asp Lys Glu Lys Val Val Lys Ile Phe Lys Glu Leu Gly Phe Gln Ser
660 665 670

Leu Leu Asp Lys Met Gly Glu His Glu Asn Glu Glu Ala Asp Glu Met
675 680 685

Pro Thr Ile Lys Phe Glu Lys Val Glu Lys Leu Ser Asp Lys Val Leu
690 695 700

Ser Glu Lys Ala Ala Leu Leu Val Glu Ile Ile Asp Glu Asn Tyr His
705 710 715 720

Thr Gly Glu Ile Ile Gly Phe Ser Ile Ala Asn Glu Asn Gly Cys Phe
725 730 735

Tyr Ile Pro Ala Glu Ile Ala Leu His Ser Lys Glu Phe Ile Glu Trp
740 745 750

Val Lys Asp Glu Thr Lys Arg Lys Val Val Tyr Asp Ala Lys Lys Ser
755 760 765

Ile Val Ala Leu Arg Trp Arg Asn Ile Asp Leu Ala Gly Ile Glu Phe
770 775 780

Asp Val Leu Ile Ala Ser Tyr Ile Leu Asn Pro Ser Glu Ser Ile Asp
785 790 795 800

Asp Ile Ala Glu Leu Ala Lys Thr Lys Asn Lys His Leu Val Gln Lys
805 810 815

Asp Glu Val Ile Tyr Gly Lys Gly Ala Lys Arg His Ile Pro Asp Glu
820 825 830

Asp Ile Leu Gly Glu His Leu Ala Arg Lys Ala Leu Ala Ile Tyr Glu
835 840 845

Leu Glu Glu Leu Leu Ile Gln Glu Leu Glu Glu Asn Glu Gln Phe His
850 855 860

Leu Phe Ser Glu Leu Glu Leu Pro Leu Ser Ala Ile Leu Ser Asp Met
865 870 875 880

Glu Thr Thr Gly Val Lys Ile Asp Val Asn Arg Leu Lys Glu Met Gly
885 890 895

Lys Glu Leu Asp Glu Gln Leu Lys Gln Leu Glu Lys Asp Ile His Arg
900 905 910

Leu Ala Gly Val Ser Phe Asn Ile Asn Ser Pro Lys Gln Leu Gly Pro
915 920 925

Ile Leu Phe Glu Lys Leu Asn Leu Pro Val Leu Lys Lys Thr Lys Thr
930 935 940

Gly Tyr Ser Thr Ser Ala Asp Val Leu Glu Lys Leu Arg Gly Gln His
945 950 955 960

Glu Ile Val Glu Lys Ile Leu His Tyr Arg Gln Leu Gly Lys Leu Gln
965 970 975

Ser Thr Tyr Ile Glu Gly Leu Leu Lys Val Val His Arg Asp Thr His
980 985 990

Lys Ile His Thr Arg Phe Asn Gln Ala Leu Thr Gln Thr Gly Arg Leu
995 1000 1005

Ser Ser Thr Asp Pro Asn Leu Gln Asn Ile Pro Ile Arg Leu Glu Glu
1010 1015 1020

Gly Arg Lys Ile Arg Gln Ala Phe Ile Pro Ser Glu Lys Asp Trp Val
1025 1030 1035 1040

Ile Phe Ala Ala Asp Tyr Ser Gln Ile Glu Leu Arg Val Leu Ala His
1045 1050 1055

Ile Ser Gly Asp Glu Lys Leu Ile Glu Ala Phe Lys Gln Asp Leu Asp
1060 1065 1070

Ile His Thr Lys Thr Ala Ile Asp Val Phe His Val Glu Glu Asp Lys
1075 1080 1085

Val Thr Ser Asn Met Arg Arg Gln Ala Lys Ala Val Asn Phe Gly Ile
1090 1095 1100

Val Tyr Gly Ile Ser Asp Tyr Gly Leu Ser Gln Asn Leu Gly Ile Thr
1105 1110 1115 1120

Arg Lys Glu Ala Gly Glu Phe Ile Lys Lys Tyr Phe Glu Ile Tyr Lys
1125 1130 1135

Gly Val Lys Glu Tyr Met Asp Gly Ile Ile Gln Glu Ala Lys Gln Lys
1140 1145 1150

Gly Tyr Val Thr Thr Leu Met Gln Arg Arg Arg Tyr Ile Pro Glu Ile
1155 1160 1165

Thr Ser Arg Asn Phe Asn Ile Arg Ser Phe Ala Glu Arg Thr Ala Met
1170 1175 1180

Asn Thr Pro Ile Gln Gly Ser Ala Ala Asp Ile Ile Lys Lys Ala Met
1185 1190 1195 1200

Ile Asp Met Ala Gln Glu Ile Glu Lys Arg Asn Leu Gln Thr Arg Leu
1205 1210 1215

Leu Leu Gln Val His Asp Glu Leu Val Phe Glu Ala Pro Lys Asp Glu
1220 1225 1230

Ile Glu Ile Leu Glu Lys Leu Val Pro Glu Val Met Glu Asn Ala Ile
1235 1240 1245

Gln Leu Lys Val Pro Leu Lys Val Asp Tyr Ser Tyr Gly Ser Thr Trp
1250 1255 1260

Tyr Asp Ala Lys Ser Ser His His His His His His
1265 1270 1275